# EUROPÄISCHE PATENTANMELDUNG

(11) **EP 4 814 153 A1**
(43) Veröffentlichungstag der Anmeldung: **30.09.2026**
(21) Anmeldenummer: 25167058.4
(22) Anmeldetag: 28.03.2025
(51) Int. Cl.: C22C 33/04, C21C 5/46, G16C 20/30, G16C 60/00, C21D 9/04, C21C 5/52, C21D 1/55

(54) **VERFAHREN UND SYSTEM ZUR HERSTELLUNG PERLITISCHER SCHIENENSTÄHLE**

(71) Anmelder: voestalpine Rail Technology GmbH, 8700 Leoben (AT); voestalpine Stahl Donawitz GmbH, 8700 Leoben-Donawitz (AT)
(72) Erfinder: Goriupp, Jürgen, 8700 Leoben (AT); Schwarzböck, Peter R., 8700 Leoben (AT); Klösch, Gerald, 8700 Leoben (AT); Karner, Peter, 8700 Leoben (AT)
(74) Vertreter: WSL Patentanwälte Partnerschaft mbB

(57) **Zusammenfassung**

Die Erfindung betrifft ein Verfahren zur Herstellung von perlitischen Schienenstählen eines auf eine Schienengüte spezifizierten Härtefaktorbereichs, welcher durch eine Ziellegierung sichergestellt wird, wobei zumindest ein primärmetallurgisches Aggregat zu einer ersten Schmelze führt, wobei aus der ersten Schmelze zumindest eine Analyse von Gehalten der Legierungselemente durchgeführt wird, wobei Ist-Werte der Schmelzanalyse an ein Steuersystem geleitet werden, wobei zwischen Einfluss- und Kompensationselementen unterschieden wird, wobei Einflusselemente Elemente sind, welche einen Einfluss auf den Härtefaktor eines Endprodukts haben, und Kompensationselemente Elemente sind, die einen Einfluss auf den Härtefaktor haben und den Einfluss von Einflusselementen kompensieren, wobei die Größe des Einflusses der Ist-Werte aller Einfluss- und Kompensationselemente auf den Härtefaktor des Endprodukts bekannt ist, wobei der zumindest eine Ist-Wert bezüglich derjenigen Einfluss- und Kompensationselemente erfasst wird, welche einen Einfluss auf den Härtefaktor des Endprodukts haben, wobei zum Härtefaktor des Endprodukts zumindest ein Kompensationselement ermittelt wird, welches den Härtefaktor einstellt, wobei eines oder mehrere Kompensationselemente in die Schmelze in einer Menge zulegiert werden, welche den Härtefaktor in einen Zielbereich bringt und den Einfluss eines oder mehrerer Einflusselemente auf den Härtefaktor kompensiert, und wobei hierfür im Steuersystem zwei Komponenten verwendet werden: ein Prognosemodell und ein Korrekturmodell, wobei mit dem Prognosemodell der Einfluss der Einfluss- und Kompensationselemente auf den Härtefaktor des Endprodukts und mit dem Korrekturmodell korrigierte Zielwerte der Kompensationselemente berechnet werden, um den Härtefaktor in einen Zielbereich zu bringen, sowie ein Steuersystem zur Durchführung des erfindungsgemäßen Verfahrens und ein Gleisteil, insbesondere eine Schiene für Schienenfahrzeuge.

## Beschreibung

Die Erfindung betrifft ein Verfahren zur Herstellung perlitischer Schienenstähle, ein System zur Herstellung perlitischer Schienenstähle und ein Gleisteil, insbesondere eine Schiene für Schienenfahrzeuge.

Weltweit wird Stahl zu 71,1% (in 2023) über die Hochofen-Route erzeugt. Dabei wird Roheisen aus Eisenerz gewonnen, indem dieses zusammen mit Reduktionsmitteln wie bspw. Koks und Zuschlagstoffen im Hochofen geschmolzen wird. Das geschmolzene Roheisen wird anschließend im Konverterverfahren (z. B. mit Sauerstoff) oder im Elektrolichtbogenofen (mit Schrott als Rohstoff) zu Rohstahl weiterverarbeitet.

Primärmetallurgische Aggregate, welche bei der Herstellung von Rohstahl Anwendung finden, können die folgenden sein: BF (Hochofen, Blast Furnace), RE-Ent-S (Roheisenentschwefelung, Hot Metal Desulfurization), BOF (Sauerstoffblaskonverter, Basic Oxygen Furnace), EAF (Elektrolichtbogenofen, Electric Arc Furnace), SAF (Elektroschmelzofen, Submerged Arc Furnace), IF (Induction Furnace, Induktionsofen), Smelter (Schmelzofen), MOE (Schmelzflusselektrolyse, Metal Oxide Electrolysis), Plasmet (Plasmareduktionsmetallurgie) usw. Anschließend wird der Rohstahl in der Sekundärmetallurgie weiterbehandelt, um die Zusammensetzung und Reinheit zu verbessern. Hierbei wird der Stahl in sekundärmetallurgischen Aggregaten wie Spülstand (Rinse Station, Purging Station), Pfannenofen (Ladle Furnace), RH-Anlagen (Ruhrstahl-Heraeus-Verfahren), VD-Anlagen (Vacuum Degassing), DETEM-Anlagen, VOD-Anlagen (Vacuum Oxygen Degassing), ASEA-SKF (Sauerstoff-Vakuumbehandlung mit Rühren), CAS-Anlagen (Composition Adjustment by Sealed Argon Bubbling), RS-V-Anlagen (Rheinstahl-Siemens-Vakuum), CAS-OB-Anlagen (Composition Adjustment by Sealed Argon Bubbling - Oxygen Blowing), Kalk-Silizium Behandlung (CaSi-Injection), Magnesium-Behandlung (Magnesium Injection), VLD-Verfahren (Vacuum Ladle Degassing) etc. behandelt, wo Zusätze (metallische sowie nicht metallische Einsatzstoffe) wie Legierungselemente und Schlackenbilder hinzugefügt werden, um die gewünschte chemische Zusammensetzung und spezifische Eigenschaften des Stahls zu erzielen.

Im Hochofen wird normalerweise kein Schrott eingesetzt, da der Prozess primär auf der Reduktion von Eisenerz basiert, um flüssiges Roheisen zu erzeugen. Schrott wird allerdings in der Stahlproduktion in anderen Prozessschritten genutzt, vor allem in Elektrolichtbogenöfen oder als Kühlmittel in Konvertern.

Das in primär metallurgischen Aggregaten erzeugte Roheisen wird anschließend in einem Sauerstoffkonverter weiterverarbeitet. In diesen Konvertern, wie dem LD-Konverter (Linz-Donawitz-Verfahren), wird flüssiges Roheisen durch Einblasen von Sauerstoff entkohlt und in Rohstahl umgewandelt. Dabei wird Schrott als Kühlmittel und zusätzliche Einsatzstoffe zugegeben. Die Zugabe von Schrott im Konverterprozess hilft, die Temperatur zu kontrollieren, da die Exothermie der Sauerstoffreaktionen die Schmelze stark erhitzt.

Ein anderer wichtiger Produktionsweg in der Stahlindustrie ist der Elektrolichtbogenofen (EAF). Hier kann bis zu 100% Schrott als Einsatzstoff verwendet werden, der durch elektrische Energie eingeschmolzen wird, um flüssigen Stahl herzustellen. Dieses Verfahren ist besonders umweltfreundlich, da zumeist die energieintensivere Reduktion nicht erforderlich ist und daher weniger CO₂-Emissionen verursacht als der Hochofenprozess.

Beim Elektrolichtbogenofenverfahren wird Stahl durch elektrische Energie erzeugt. Dabei wird ein elektrischer Lichtbogen zwischen Graphitelektroden und dem Schrottmaterial im Ofen erzeugt, wodurch hohe Temperaturen (bis zu 3.500 °C im Bereich des Lichtbogens) entstehen, die den Schrott schmelzen und ihn so zu flüssigem Rohstahl verarbeiten.

Bei der Herstellung von Rohstahl werden Einsatzstoffe, wie beispielsweise Schrott, verwendet. Es wird zwischen metallischen und nichtmetallischen Einsatzstoffen unterschieden. Dies sind zum Beispiel Eisenerz, Reduktionsmittel, Schlackenbildner, Eisenschwamm in Form von direkt reduziertem Eisen DRI bzw. heiß brikettiertem Eisen HBI, Desoxidationsmittel, Legierungsmittel und Schrott.

Die Verwendung von Schrott als Rohmaterial für Stahl bietet mehrere Vorteile. Das Einschmelzen von Schrott benötigt lediglich die Energie zum Erwärmen bzw. Schmelzen und, da zumeist die energieintensive Reduktion von vorgelagerten Prozessen durchgeführt wird, weniger Energie als die Primärproduktion von Rohstahl aus Eisenerz. Außerdem reduziert Schrottrecycling die CO₂-Emissionen und den Bedarf an neuen Rohstoffen, was die Umweltbelastung senkt. Da Stahl immer wieder recycelt werden kann, ist die Nutzung von Schrott ein wichtiger Beitrag zur Kreislaufwirtschaft in der Stahlindustrie.

Obwohl die Schrottverwendung im Elektrolichtbogenofen (EAF) viele Vorteile mit sich bringt, gibt es auch einige Nachteile und Herausforderungen, die berücksichtigt werden müssen.

Die erste Herausforderung bei Verwendung von Schrott ist die schwankende Schrottqualität. Der Schrott, der im EAF verwendet wird, ist oft inhomogen und kann Verunreinigungen enthalten, die sich negativ auf die Stahlqualität auswirken. Zum Beispiel können unerwünschte Elemente wie Kupfer, Molybdän, Zinn, Nickel und Chrom im Schrott vorkommen, die im Rohstahl schwer zu entfernen sind und für bestimmte Anwendungen negative Auswirkungen in der Sekundärmetallurgie nachgeschalteten Anlagen und Eigenschaften des Endproduktes haben. Verunreinigungen können den EAF-Prozess komplizierter und teurer machen, da zusätzliche Aufbereitungsschritte oder Sortierungen erforderlich sein können.

Die zweite große Herausforderung betrifft die Kontrolle der chemischen Legierungszusammensetzung. Da Schrott unterschiedliche Legierungen und Metallzusammensetzungen haben kann, kann es schwer sein, die chemische Zusammensetzung des Endprodukts präzise zu steuern. Die Zugabe von Legierungselementen im EAF wird daher komplizierter, und es sind oft zusätzliche Anpassungen nötig, um die gewünschte Stahlspezifikation zu erreichen.

Aufgrund der möglichen Verunreinigungen und der schwankenden Qualität des Schrotts ist es schwer, mit dem EAF-Verfahren besonders niedrige Gehalte (< 0,005 Gew.-%) an Elementen wie Kupfer, Zinn, Zink und Molybdän zu gewährleisten, die für bestimmte spezialisierte Anwendungen erforderlich sind. In solchen Fällen ist die Nutzung von Primärmaterialien (bspw. Roheisen, pig iron, flüssiges Roheisen, hot metal, DRI, HBI) oder sehr hochwertigem, sortenreinem Schrott notwendig, was die Kosten erhöht.

Neben der chemischen Zusammensetzung, welche in einem Elektrolichtbogenofenprozess einsatzstoffbedingten Schwankungen unterliegt und deshalb oft in vorbestimmten Toleranzgrenzen gehalten wird, sind auch die Auswirkungen einzelner Elemente auf die Eigenschaften des Endprodukts zu berücksichtigen.

Insbesondere bei einer nachfolgenden Wärmebehandlung oder Umformprozessen, wie beispielsweise Walzen, werden die werkstoffkundlichen Eigenschaften des Stahls zusätzlich gesteuert.

Es sind bereits einige Lösungen bekannt, welche die unerwünschten Begleitelemente in der Legierungszusammensetzung berücksichtigen oder zusätzliche Anpassungen der Legierungselemente vorsehen.

Es ist üblich, fest definierte Zielbereiche für eine Stahlgüte festzulegen, welche den minimalen und den maximalen Wert je Legierungselement vorsehen. Für Begleitelemente, welche nicht bewusst legiert aber in den Einsatzstoffen in unbekanntem Gehalt enthalten sind, existieren Maximalgehalte, welche sicherstellen sollen, dass sofern diese Gehalte unterschritten werden, die Werkstoffeigenschaften innerhalb des gewünschten Bereiches gehalten werden. Auf den tatsächlichen Gehalt dieser Begleitelemente wird in der Flüssigphase allerdings nicht reagiert.

Dabei ist von Nachteil, dass niedrige Maximalgehalte der Begleitelemente die Verwendung von Einsatzstoffen mit geringen Gehalten dieser Elemente erzwingen. Dies wird in erster Linie durch einen hohen Anteil an aus Primärstoffen erzeugten Einsatzstoffen erreicht.

Aus der EP 3 956 481 B1 ist ein computergestütztes Verfahren zur Überwachung des Stahlherstellungsprozesses in einem Konverter bekannt. Dabei werden verschiedene Materialien mit spezifischen Eigenschaften in den Konverter eingebracht, um flüssigen Rohstahl und Schlacke zu produzieren. Bei dem Verfahren werden zuerst die gewünschten Eigenschaften des zu produzierenden flüssigen Rohstahls und der Schlacke festgelegt. Dann werden die erforderlichen Mengen jedes Materials, die in den Konverter eingebracht werden müssen, berechnet, um die definierten Zielmerkmale des Rohstahls und der Schlacke zu erreichen. Die berechneten Materialmengen werden an den Bediener oder an automatische Ladesysteme übermittelt und der Konverter wird entsprechend beladen.

Somit wird hier das Beladen des Konverters mit verschiedenen Ausgangsstoffen beschrieben. Nachteilig ist, dass dabei lediglich die Zusammensetzung der im Konverter erzeugten Schmelze kontrolliert wird. Eine nachträgliche Einflussnahme auf die Legierungszusammensetzung wird nicht spezifiziert.

Aus der DE 10 2021 211 320 A1 ist ein Verfahren zur Steuerung und Regelung einer Produktionsanlage für Walzprodukte aus metallischen Legierungen wie Stahl, Eisen oder Aluminium bekannt. Ziel des Verfahrens ist es, den Einsatz von Rohstoffen zu optimieren und die Produktionskosten zu senken. Anfangs werden für jeden Auftrag spezifische Sollwerte für Material-, Oberflächen- und geometrische Eigenschaften des Endprodukts sowie zulässige chemische Zusammensetzungen mit definierten Toleranzbereichen festgelegt. Durch Anwendung von Prozessmodellen werden für jeden Auftrag prädiktive Ist-Werte der Produkteigenschaften berechnet und mit den Sollwerten verglichen. Nur Zusammensetzungen, die innerhalb der spezifizierten Toleranzen liegen, werden ausgewählt. Für jede ausgewählte chemische Zusammensetzung werden Kostenparameter wie Legierungs-, Einsatzstoff-, Energie-, Eisen-, Zuschlagsstoff- und CO₂-Kosten ermittelt. Diese werden in Form von Strafpunkten oder einer Straffunktion quantifiziert. Anschließend wird die Schnittmenge der zulässigen chemischen Zusammensetzungen für verschiedene Kombinationen von Produktionsaufträgen, die in einer gemeinsamen Charge erschmolzen werden sollen, ermittelt.

Nachteilig dabei ist, dass hier Toleranzbereiche für jede Zusammensetzung ermittelt werden. Eine genaue Steuerung der Zusammensetzung ist mit diesem Modell nicht möglich.

Aus der EP 4 183 498 A1 ist ein KI-basiertes Prognosemodell bekannt, mit welchem sich die mechanischen Eigenschaften aus einigen vorher gemessenen Ausgangswerten, wie beispielsweise der Zusammensetzung der Schmelze, vorhersagen lassen. Um einen Einfluss auf die mechanischen Eigenschaften zu nehmen, wird als Output eine Anpassung der Herstellungsparameter, wie beispielsweise Vorwalzverhältnis, Fertigwalzverhältnis, Walzstarttemperatur, Startzeit der Kühlung, Abkühlungsrate oder Liniengeschwindigkeit vorgeschlagen.

Das Modell ist auf spezifische Produktionslinien und -prozesse optimiert. Wenn die Produktionsbedingungen oder Anforderungen schnell geändert werden müssen, kann das System Schwierigkeiten haben, sich anzupassen.

Perlitische Schienenstähle sind eine spezielle Stahlgruppe, die sich durch eine perlitische Mikrostruktur auszeichnet, die hohe Festigkeit und Verschleißfestigkeit bietet. Diese Stähle sind besonders für den Einsatz in Eisenbahnschienen und Schwellen geeignet, da sie den hohen Belastungen standhalten, die durch Zugverkehr und hohe Achslasten entstehen.

Die Herstellung dieser Stähle erfolgt in der Regel über folgende Schritte: Stahlerzeugung, Formgebung und Abkühlung sowie gegebenenfalls eine Wärmebehandlung.

Perlitische Schienenstähle werden meist in einem Elektrolichtbogenofen oder einem Sauerstoffkonverter hergestellt. Die Hauptbestandteile neben Eisen sind Kohlenstoff (ca. 0,7-0,8%) und Mangan (ca. 1%), wobei weitere Elemente wie Silizium, Chrom und Vanadium hinzugefügt werden können, um spezifische Eigenschaften zu erzielen.

Nach dem Gießen und Walzen der Schienen wird die Abkühlung genau kontrolliert, um eine feinkörnige perlitische Mikrostruktur zu erzeugen. Die perlitische Struktur besteht aus abwechselnden Schichten von Ferrit (weich und duktil) und Zementit (hart und spröde), was eine hervorragende Kombination von Härte und Zähigkeit bewirkt.

In bestimmten Anwendungen kann eine Wärmebehandlung durchgeführt werden, um die Verschleißfestigkeit weiter zu verbessern. Das "Kopf-Härten" ist ein Verfahren, das speziell den Schienenkopf behandelt, um die Härte in diesem stark belasteten Bereich zu erhöhen.

Die perlitische Mikrostruktur in diesen Stählen entsteht u.a. durch kontrollierte Abkühlung und ist entscheidend für ihre werkstoffkundlichen Eigenschaften.

Ferner zeichnen sich perlitische Schienenstähle durch eine hohe Verschleißbeständigkeit aus, was besonders wichtig ist, da die Schienenoberfläche mechanischen Belastungen und Abrieb ausgesetzt ist.

Außerdem verleiht die Kombination von Ferrit und Zementit den Stählen die nötige Zähigkeit, um den hohen dynamischen Belastungen im Schienenverkehr standzuhalten.

Perlitische Schienenstähle werden in verschiedenen Bereichen des Schienenverkehrs, wie beispielsweise Hochgeschwindigkeitsstrecken, Schwerlastverkehr oder Kurvengleise, eingesetzt.

Somit sind perlitische Schienenstähle speziell entwickelte Materialien, die durch ihre Zusammensetzung und kontrollierte Wärmebehandlung eine ideale Mikrostruktur für den Schienenverkehr bieten. Die Vorteile ihrer perlitischen Struktur - hohe Härte, Festigkeit und Zähigkeit - machen sie zu einer langlebigen und robusten Wahl für Schienen, die in anspruchsvollen Verkehrsumgebungen eingesetzt werden.

Aus dem Grund ist die Einhaltung konstanter Produkteigenschaften von großer Bedeutung.

Aufgabe der Erfindung ist es, ein Verfahren zur Herstellung perlitischer Schienenstähle zur Verfügung zu stellen, mit welchem konstante Produkteigenschaften auch bei größeren Schwankungen der Begleitelemente gewährleistet werden.

Die Aufgabe wird mit einem Verfahren mit den Merkmalen des Anspruchs 1 gelöst.

Vorteilhafte Weiterbildungen sind in den Unteransprüchen gekennzeichnet.

Ferner ist es Aufgabe der Erfindung, ein System zur Herstellung perlitischer Schienenstähle zur Verfügung zu stellen, mit welchem es gelingt, konstante Produkteigenschaften auch bei größeren Schwankungen der Begleitelemente zu gewährleisten.

Die Aufgabe wird mit einem System mit den Merkmalen des Anspruchs 18 gelöst.

Vorteilhafte Weiterbildungen sind in den Unteransprüchen gekennzeichnet.

Ferner ist es Aufgabe der Erfindung, ein Gleisteil, insbesondere eine Schiene für Schienenfahrzeuge zur Verfügung zu stellen.

Diese Aufgabe wird mit einem Gleisteil mit den Merkmalen des Anspruchs 25 gelöst.

Es handelt sich um ein erfindungsgemäßes Konzept eines dynamischen Legierens. Dabei wird eine Adaption der Elementgrenzen für Legierungselemente in der Flüssigphase durchgeführt.

Durch eine Analyse der Schmelze werden Ist-Werte der Elementgehalte bestimmt. Diese Ist-Werte werden in ein für einen spezifizierten Schienenstahl passendes, datengetriebenes Prognosemodell eingespeist, sodass der Einfluss der jeweiligen Elemente auf eine werkstoffkundliche, insbesondere mechanische Eigenschaft modelliert und, wenn dies notwendig ist, um die werkstoffkundliche, insbesondere mechanische Eigenschaft in einen gewünschten Zielbereich zu bringen, durch ein Korrekturmodell eine Reaktion in Form von adaptierten Legierungselementgrenzen aufgefunden wird und diese dem Legierungsrechner als adaptierte Zielwerte zugeführt werden. Der Legierungsrechner benutzt die adaptierten Legierungselementgrenzen zur Festlegung der Massen an Einsatzstoffen.

Die Erfinder haben erkannt, dass bei der dynamischen Korrektur der Elementgehalte zwischen zwei definierten Elementarten unterschieden werden soll. Es handelt sich hierbei um Einfluss- und Kompensationselemente.

Bei der Stahlherstellung werden beim Herstellungsprozess verschiedene Einsatzstoffe verwendet. Dies sind zum Beispiel Eisenerz, Eisenschwamm in Form von direkt reduziertem Eisen (DRI) bzw. heiß brikettiertem Eisen (HBI) und Schrott. Über diese Einsatzstoffe wird natürlich nicht nur Eisen eingebracht, sondern auch sogenannte Begleitelemente und Spurenelemente.

Begleitelemente sind üblicherweise nicht vermeidbare Elemente, wie beispielsweise Si, Mn, S, P, O, N und H, und werden im Verlauf eines nachfolgenden metallurgischen Prozesses entfernt, sofern das möglich ist. Ist ein Entfernen nicht möglich, müssen das jeweilige Begleitelement und sein Einfluss bei metallurgischen Prozessen berücksichtigt werden.

Für jede Stahlsorte, welche durch die chemische Zusammensetzung der Legierung und die Herstellroute definiert wird, existiert eine definierte, aus praktischen Erfahrungswerten resultierende Begleitelementklasse, d.h. es müssen bestimmte Obergrenzen der Gehalte der Begleitelemente eingehalten werden.

Bei Spurenelementen handelt es sich um explizit störende Begleitelemente (z.B. As und Sb), welche in sehr geringen Mengen vorliegen. Diese werden im Zuge des (hier durchgeführten dynamischen) Legierens nicht berücksichtigt.

Legierungselemente sind Elemente, welche gezielt zugesetzt werden, um dem Stahl gewünschte, werkstoffkundliche Eigenschaften zu verleihen. Solche Eigenschaften können mechanische Eigenschaften wie Härte, Zugfestigkeit, Zähigkeit und weitere Eigenschaften sein, aber auch chemische Eigenschaften wie Widerstand gegen Korrosion oder Wasserstoffversprödung und weitere.

Bei Einsatz von Einsatzstoffen, insbesondere metallischen Einsatzstoffen und nicht metallischen Einsatzstoffen, muss beachtet werden, dass sie für sich gesehen Begleitelemente aus ihrer Rohstoffhistorie und Legierungselemente aus ihrer metallurgischen Historie enthalten.

Wird nun beispielsweise Schrott als Einsatzstoff in der Stahlherstellung verwendet, sind seine Bestandteile, bis auf Eisen, für das Produkt, was unter Einsatz des Schrotts erzeugt werden soll, Begleit- und Spurenelemente.

Deren Anwesenheit muss also berücksichtigt werden, da sie (als ehemalige Legierungselemente) aus dem Produkt über metallurgische Prozesse entweder nur mit großem Aufwand oder nicht entfernbar sind. Jedoch kann ihre Wirkung erfindungsgemäß berücksichtigt und kompensiert werden.

Im klassischen Herstellungsprozess wird von einem Produkt (Stahl) ausgegangen, welches entsprechend den Marktanforderungen bestimmte werkstoffkundliche Eigenschaften aufweist. Auf dieses Anforderungsprofil hin, wird es chemisch/metallurgisch eingestellt. Ein solches Produkt ist also eine Legierung, wobei die Legierungsbestandteile durch einen definierten Gehalt an Obergrenzen oder mit Unter- und Obergrenzen bestimmt sind. Von einer bestimmten Legierung ist somit bekannt, welche Eigenschaften sie haben kann.

Wird nun aufgrund geänderter Bedingungen, der Einsatz von Einsatzstoffen wie bspw. Schrott erheblich erhöht, wird das Produkt folgerichtig mit einer höheren Menge von Begleitelementen befrachtet. Diese Begleitelemente haben (als "ehemalige" Legierungselemente) einen Einfluss zum Beispiel auf werkstoffkundliche Eigenschaften des Produkts.

Insofern muss dies bei dem Zulegieren berücksichtigt werden. Hierbei können die folgenden Fälle eintreten.
A) Beispielsweise wird für Element A durch den Einsatzstoff ein Anteil eingebracht, der unterhalb des gewünschten Gehalts liegt. In diesem Fall muss ggf. weniger Element A zulegiert werden.
B) Für Element A wird über den Einsatzstoff ein Gehalt eingebracht, der bereits im Zielfenster liegt, es muss nichts mehr zulegiert werden.
C) Es wird mehr Element A als gewünscht eingebracht. Der Gehalt des Elements A kann nur unter erhöhtem Aufwand metallurgisch verringert werden. Dementsprechend muss die Wirkung des Elements A kompensiert werden.

Das obige Beispiel wird dann komplexer, wenn mehrere Begleitelemente oder Legierungselemente für bestimmte Eigenschaften zusammenspielen.

Hier setzt die Erfindung dadurch an, dass zu den Begleitelementen, die einen Einfluss auf eine oder mehrere gewünschte, werkstoffkundliche Eigenschaften haben (nachfolgend Einflusselemente genannt), Legierungselemente ausgewählt werden, die den Einfluss des Einflusselements auf einen gewünschten Einflussumfang kompensieren können (nachfolgend Kompensationselemente genannt).

Kompensationselemente sind Elemente, die bestenfalls ohnehin reguläre Legierungselemente der Ziellegierung sind, sodass im Rahmen der qualitativen gewünschten Legierungszusammensetzung lediglich quantitative Anpassungen erfolgen.

Ein einfaches Beispiel soll dies deutlich machen. Sind neben Eisen in einer gewünschten Stahllegierung die Elemente A, B und C enthalten und ist das Element A in einer Menge vorhanden, die die gewünschte Menge übersteigt, und ist C ein Element, welches den Einfluss von A auf eine definierte Zielgröße verringert, wird C in einer Menge zulegiert, welche zusätzlich zur eigenen Wirkung noch die Wirkung von A kompensiert.

Ein weiteres Beispiel ist, wenn Element A durch die Einsatzstoffe in einer Menge vorhanden ist, die die gewünschte Menge überschreitet. Wenn das Element C einen vergleichbaren Einfluss auf eine gewünschte Eigenschaft hat wie A, jedoch in der Legierung wenig oder nur in Spuren vorhanden ist, wird hiervon nur so viel zugesetzt, dass die addierte Wirkung von A und C gemeinsam den Zielwert der gewünschten Eigenschaft zur Folge haben. D.h. in diesem Fall, dass weniger C zugesetzt wird als normalerweise zugesetzt würde, um den zu hohen A-Gehalt zu kompensieren.

Kompensation im Sinne der Erfindung bedeutet somit, dass eine Anpassung der Ziellegierungszusammensetzung nach oben oder unten stattfinden kann.

B ist in diesem Fall z.B. ein Element, welches auf die definierte Zielgröße, beispielsweise Festigkeit, keinen Einfluss hat.

Insgesamt kann durch dieses erfindungsgemäße Vorgehen, die Einsatzstoffvorauswahl gröber erfolgen.

Eingangskenngrößen in das Prognosemodell sind alle Elemente, die in der Flüssigphase enthalten und analysiert worden sind.

Ausgangsgrößen sind adaptierte Elementgrenzen für Elemente, welche in der Flüssigphase legiert werden. Es handelt sich hierbei um die sogenannten Kompensationselemente, wie beispielsweise C, Si, Mn, Cr. Kompensationselemente sind somit Elemente, welche in der Flüssigphase zulegiert werden, um den Einfluss der Einflusselemente auszugleichen, damit die Soll-Eigenschaften der Endlegierung erreicht werden.

Sowohl die Einfluss- als auch die Kompensationselemente sind Elemente, welche durch Zugabe von Einsatzstoffen in die Schmelze eingebracht werden.

Dabei handelt es sich um Begleitelemente, die im Eisen oder Stahl unvermeidlich vorkommen und in geringer Menge enthalten sein können. Sie können aus den verwendeten Rohstoffen, wie Erz oder Schrott, stammen oder bei der Verarbeitung in die Schmelze eingebracht werden. Diese Begleitelemente beeinflussen die Eigenschaften des Stahls und können je nach Gehalt positive oder negative Effekte auf Festigkeit, Korrosionsbeständigkeit, Zähigkeit und Verformbarkeit haben.

In der Stahlherstellung wird "Schrott" als recycelter, wiederverwendbarer metallischer Sekundärstoff bezeichnet, der als Einsatzstoff zur Produktion von neuem Stahl dienen. Schrott besteht hauptsächlich aus Eisenbasislegierung und wird in verschiedenen Qualitäten und Sorten klassifiziert, je nach Reinheit, Form, Größe und chemischer Zusammensetzung.

Das vorliegende Verfahren, bei dem Einsatzstoffe eingesetzt werden, betrifft die Herstellung perlitischer Schienenstähle.

Das Konzept beruht darauf, ein Endprodukt einer bestimmten Stahlsorte zu erzeugen, bei denen sich werkstoffkundliche, insbesondere mechanische Eigenschaften, wie beispielsweise Streckgrenze und Zugfestigkeit, sowie auch andere Kenngrößen wie z.B. das Kohlenstoffäquivalent (CAE), trotzt erhöhter Gehalte, welche durch die metallischen Einsatzstoffen im primärmetallurgischen Aggregat eingebracht werden, gar nicht oder nur möglichst geringfügig ändern und zwar im Vergleich zu den Festigkeitseigenschaften und anderen Kenngrößen, die sich einstellen, wenn bei der Herstellung der Schmelzen kleinere Einsatzstoffanteile eingesetzt werden und damit geringere Anteile an unerwünschten Begleitelementen eingebracht werden (z.B. beim LD-Verfahren). Dies stellt auch sicher, dass auf die Stahlherstellung folgende Nachbehandlungsschritte nicht geändert werden müssen.

Dies geschieht durch Adaption eines oder mehrere Legierungselemente, und wird nachfolgend als dynamisches Legieren bezeichnet. Die Adaption erfolgt durch Anwendung eines Prognosemodells, welches zunächst aufgrund von Analyse-Ist-Werten der Legierungszusammensetzung eines primärmetallurgischen Aggregats die erwartbare werkstoffkundliche, insbesondere mechanische Eigenschaft modelliert. Sollte diese außerhalb des durch die gewünschte Schienenstahlgüte spezifizierten Zielbereichs liegen, werden mittels eines Korrekturmodells korrigierte Legierungselementgehalte berechnet, sodass die spezifizierten Anforderungen an die werkstoffkundliche, insbesondere mechanische Eigenschaft erfüllt werden können. Die Adaption erfolgt somit durch Anwendung eines Prognosemodells für werkstoffkundliche Eigenschaften kombiniert mit einem Korrekturmodell.

Für Schienenstähle relevante werkstoffkundliche Eigenschaften sind insbesondere die Härte, Zugfestigkeit, Dehnung sowie metallographischen Ausprägungen, insbesondere Gefüge. Mit dem Prognosemodell wird die Wirkung der Elemente als Härtefaktor (HF) berechnet. Der Härtefaktor beschreibt einen Kennwert, welcher über Nebenbedingungen mit werkstoffkundlichen Eigenschaften, insbesondere Härte, Zugfestigkeit, Dehnung sowie metallographischen Ausprägungen, insbesondere Gefüge, korreliert.

Der Härtefaktor kann Werte zwischen 0 % und 100 % einnehmen, wobei 0% einer Härte von 200 HB und 100 % einer technisch machbaren Härte entspricht.

Nach EN 13674 liegt diese Härte derzeit bei 440 HB.

Die tatsächlich technisch machbare Härte kann derzeit bis 480 HB angenommen werden.

Die vorliegenden Beispiele beziehen sich auf eine Härteobergrenze von 400 HB, werden höhere Härtewerte eingerechnet ändern sich die Faktoren des angegebenen Formalismus entsprechend.

Zum Härtefaktor (HF) wird zumindest ein Kompensationselement ermittelt, welches den Härtefaktor einstellt. Die korrigierten Soll-Werte bzw. der Zielbereich des zumindest einen Kompensationselements wird mithilfe des Korrekturmodells berechnet und das zumindest eine Kompensationselement wird in einer Menge zulegiert, welche den Härtefaktor in den Zielbereich bringt.

Nach der Zugabe von Kompensationselementen kann eine weitere Analyse getätigt werden. Der Ist-Wert der Legierungszusammensetzung wird kontrolliert und bei Bedarf erneut dynamisch mithilfe des Prognose- und Korrekturmodells korrigiert.

Für die Erzeugung des Prognose- und/oder Korrekturmodells können unterschiedliche Machine-Learning-Modelle herangezogen werden, beispielsweise lineare Regressionsmodelle, polynomiale Regressionsmodelle, Decision-Tree-Based Regressionsmodelle, Random-Forest-Regressionsmodelle, Nearest-Neighbour-Modelle, Neuronal-Network-Modelle, Support-Vector-Machine-Modelle, ADA-Boost-Modelle, Regression-Boost-Modelle, HIST-Boost-Modelle, XGBOOST-Modelle, Generalized-Additive-Modelle, Symbolic-Regression-Modelle.

Diese Modelle sind datengetrieben. Als Datenbasis für die chemischen und mechanischen Werte können beispielsweise datenbankbasierte Abfragen herangezogen werden. Auch Prozessparameter, insbesondere Wärmebehandlungstemperatur und Haltzeiten, aus entsprechenden datenbankbasierten Abfragen können genutzt werden. Ferner können Datensätze auch durch Simulationsdaten erweitert werden, um den Einfluss höherer Einflusselementfracht abzubilden. Es findet eine Aufteilung der Datenbasis in Trainings- und Validierungsdaten statt, beispielsweise werden 80% der Daten zu Trainingszwecken und 20% der Daten zu Validierungszwecken verwendet.

Zur Verbesserung der Genauigkeit der Modelle könnten zusätzlich zur chemischen Zusammensetzung weitere wichtige Prozessparameter, wie beispielsweise die Temperaturführung des Walz- und Abkühlprozesses, in den Modellen berücksichtigt werden. Zudem ist es hierfür sinnvoll hinsichtlich einer bestimmten werkstoffkundlichen, insbesondere mechanischen Eigenschaft die relevantesten Wirkparameter und gegebenenfalls vernachlässigbare Parameter zu identifizieren.

Rein datengetriebene Modelle eignen sich gut für eine Vorhersage im durch die Trainingsdaten bestimmten Datenraum. Die Vorhersage mag sich jedoch verschlechtern, sollte signifikant von diesem bekannten Datenraum abgewichen werden, d.h. wenn eine stark abweichende Legierungszusammensetzung in die Berechnung der werkstoffkundlichen, insbesondere mechanischen Eigenschaft mittels des Prognose- und/oder Korrekturmodells einfließt. Kleine Trainingsdatensätze leiden zudem gegebenenfalls unter großer Streuung, sodass die Vorhersagegenauigkeit beeinträchtigt werden kann. Dieser Effekt kann durch größere Trainingsdatensätze minimiert werden.

Um für die Modellierung des Härtefaktors das Modell mit der höchsten Genauigkeit zu identifizieren, werden die durch Analyse der Schmelze bestimmten Ist-Werte in mögliche Modelle (siehe obige Aufzählung) eingespeist. Das Prognosemodell und/oder Korrekturmodell mit der höchsten Genauigkeit, welches sich durch die geringste Abweichung/den geringsten Wert der Quadratwurzel des mittleren quadratischen Fehlers (root mean square error, RMSE) des Härtefaktors auszeichnet, wird schließlich zur Modellierung des Härtefaktors herangezogen. Bei Betrachtung mehrerer werkstoffkundlicher, insbesondere mechanischer Eigenschaften wird der Mittelwert und/oder die Summe der Quadratwurzeln der mittleren quadratischen Fehler der werkstoffkundlichen, insbesondere mechanischen Eigenschaften herangezogen.

Der Einfluss der einzelnen Elemente auf die werkstoffkundlichen Eigenschaften der Legierung ist wie folgt.

Kupfer wirkt in Schienen verfestigend und härtesteigernd und vermindert zugleich die Warmumformbarkeit und Bruchdehnung. Ebenfalls können selektive Kupferanreicherungen an der Schienenstahloberfläche beobachtet werden. Kupfer kann die Festigkeit des Stahles durch Ausscheidungen bei >0.8%Cu erhöhen. Darunter ist die festigkeitssteigernde Wirkung des Kupfers auf Mischkristallverfestigung rückzuführen. Durch niedrigschmelzende Phasen an den Korngrenzen kann sich somit die Warmumformbarkeit wesentlich verschlechtern. Dies führt in weiterer Folge zu Rissen am Endprodukt, welche im schlimmsten Fall, im Gleisbetrieb zu Schienenbrüche führen können. Deshalb ist es vorteilhaft, wenn ein Kupfergehalt von 0,001-0,4 Gew.-%, vorzugsweise 0,001-0,38 Gew.-%, besonders bevorzugt 0,001-0,35 Gew.-%, weiterhin besonders bevorzugt 0,001-0,3 Gew.-% gewählt wird.

Nickel erhöht die Zähigkeit von Stahl, steigert die Härte und Festigkeit. Darüber hinaus erhöht Nickel die Löslichkeit von Kupfer im Schienenstahl. Deshalb ist es vorteilhaft, wenn ein Nickelgehalt von 0,001-0,4 Gew.-%, vorzugsweise 0,001-0,38 Gew.-%, bevorzugt 0,001-0,35 Gew.-%, besonders bevorzugt 0,001-0,3 Gew.-% gewählt wird.

Schon geringe Mengen von Molybdän steigern die Härtbarkeit des Stahles wesentlich. Bei zu hohen Analyse-Schwankungen wird die Gefügeausprägung zunehmend instabil. Neben bruchauslösenden Martensitphasen im Seigerungsbereich können dadurch unerwünschte Fremdgefügebestandteile entstehen, welche zur verstärkten Oberflächenermüdung führen können. Darüber hinaus wird die Schweißbarkeit des Stahles mit steigenden Molybdängehalt vermindert. Der Einfluss von Molybdän auf das Umwandlungsverhalten erhöht bei größeren Analysenschwankungen auch die Prozessunsicherheit der dem Walzprozess nachgeschalteten Wärmebehandlungsanlage, da bei der beschleunigten Abkühlung die Ausprägung unerwünschter Mischphasen am Schienenkopfumfang möglich werden. Entsprechende betriebliche Erfahrungen zeigten eindeutig, dass bei einer Überschreitung kritischer Werte zunehmend mit Mischphasenanteile zu rechnen ist. Molybdän fördert Unterkühlung und wirkt somit positiv auf die Lamellenfeinheit von perlitischen Schienenstählen. Deshalb ist es vorteilhaft, wenn ein Molybdängehalt von 0,001-0,06 Gew.-%, vorzugsweise 0,001-0,058-Gew.-%, besonders bevorzugt 0,001-0,055 Gew.-% gewählt wird.

Zinn wirkt verfestigend und härtesteigernd, vermindert zugleich die Bruchdehnung und neigt zu stärkeren Seigerungen, wodurch die Gefügestabilität beeinflusst wird. Zinn wirkt darüber hinaus oberflächenaktiv und verschlechtert die Warmumformbarkeit. Deshalb ist es vorteilhaft, wenn ein Zinngehalt von 0,001-0,03 Gew.-%, vorzugsweise 0,001-0,028 Gew.-%, bevorzugt 0,001-0,026 Gew.-%, besonders bevorzugt 0,001-0,024 Gew.-% gewählt wird.

Kohlenstoff bildet Karbide mit den unterschiedlichsten Elementen, daher ist die Härte direkt proportional zum Kohlenstoffgehalt. Bei einem hypereutektoiden Kohlenstoffgehalt muss die Wärmebehandlung so eingestellt werden, dass die Bildung von Sekundärzementit an den Korngrenzen verhindert wird. Kohlenstoff dient in Schienenstählen über die Karbide als Verschleißträger und hat hohe festigkeitssteigernde Eigenschaften bei gleichzeitiger Verschlechterung der Bruchdehnung und Schweißbarkeit. Deshalb ist es vorteilhaft, wenn ein Kohlenstoffgehalt von 0,38-1,4 Gew.-%, vorzugsweise 0,38-1,39 Gew.-%, bevorzugt 0,38-1,35 Gew.-%, besonders bevorzugt 0,038-1,3 Gew.-%, weiterhin besonders bevorzugt 0,38-1,25 Gew.-% gewählt wird.

Silizium wirkt als Mischkristallhärter im Ferrit und erhöht so die Härte des Perlits, ist aber für das Verschleißverhalten nicht relevant, da in der verformten Zone im Bereich des Rad-Schiene-Kontaktes der Zementit im Perlit der bestimmende Parameter ist. Silizium senkt stark die Umwandlungstemperatur und begünstigt dadurch ein vollperlitisches Gefüge mit übereutektoiden Kohlenstoffgehalten bei naturharter Abkühlung sowie bei wärmebehandelten Schienen. Darüber hinaus behindert es die Zementitbildung und hemmt somit die Ausscheidung von Korngrenzenzementit bei hypereutektoiden Stählen. Silizium hat außerdem hohen Einfluss auf die elektrische Leitfähigkeit. Deshalb ist es vorteilhaft, wenn ein Siliziumgehalt von 0,1-1,2 Gew.-%, vorzugsweise 0,1-1,15 Gew.-%, bevorzugt 0,1-1,10 Gew.%, besonders bevorzugt 0,1-1,05 Gew.-%, weiterhin besonders bevorzugt 0,1-1,0 Gew.-% gewählt wird.

Mangan bindet Schwefel zu Mangansulfiden. Mangan erhöht die Durchhärtbarkeit und senkt daher die kritische Abkühlgeschwindigkeit. Dadurch können bei geringeren Abkühlgeschwindigkeiten höhere Härten erreicht werden. Mangan ist ein starker Austenitstabilisator. In Kombination mit höheren Kohlenstoff- und Chromgehalten besteht aber die Gefahr der Bainitbildung. Mangan neigt ebenfalls zu stärkeren Seigerungen. Deshalb ist es vorteilhaft, wenn ein Mangangehalt von 0,2-1,8 Gew.-%, vorzugsweise 0,2-1,78 Gew.-%, bevorzugt 0,2-1,75 Gew.-%, besonders bevorzugt 0,2-1,7 Gew.-%, weiterhin besonders bevorzugt 0,2-1,65 Gew.-% gewählt wird.

Chrom erhöht die Durchhärtbarkeit und senkt die kritische Abkühlgeschwindigkeit. Genauso wie bei Mangan gilt auch für Chrom, dass in Kombination mit höheren Kohlenstoff- und Mangangehalten die Gefahr der Bainitbildung entsteht. Die durchhärtende Wirkung von Chrom ist deutlich stärker als bei Mangan. Darüber hinaus hat Chrom einen negativen Einfluss auf die Schweißbarkeit. Die Bildung von Chromkarbiden ist ebenfalls möglich. Deshalb ist es vorteilhaft, wenn ein Chromgehalt von 0,001-1,5 Gew.-%, vorzugsweise 0,001-1,45 Gew.-%, bevorzugt 0,001-1,4 Gew.-%, besonders bevorzugt 0,001-1,35 Gew.-%, weiterhin besonders bevorzugt 0,001-1,3 Gew.-% gewählt wird.

Vanadium wirkt allgemein kornfeinend und festigkeitssteigernd. Grenzen sind hierbei hauptsächlich ökonomischer Natur auf Basis der Volatilität des eingesetzten Ferrovanadiums. Deshalb ist es vorteilhaft, wenn ein Vanadiumgehalt von 0,001-0,3 Gew.-%, vorzugsweise 0,001-0,30 Gew.-%, bevorzugt 0,001-0,28 Gew.-%, besonders bevorzugt 0,001-0,26 Gew.%, weiterhin besonders bevorzugt 0,001-0,24 Gew.-% gewählt wird.

Die Elemente C, Si, Mn, Cr und V werden als Kompensationselemente betrachtet.

Es ist zu beachten, dass die schädliche Wirkung von Einflusselementen vor allem in Kombination untereinander als kritisch zu sehen ist, wobei deren Ursprung in entsprechenden Einsatzmaterialverunreinigung (beim Einsatzmix zur Erstellung der Schmelze) begründet ist. Bei Verwendung von Schmelztechnologien mit erhöhten Einsatzmaterialmengen können somit durch die unweigerlich erhöhte Einflusselementfracht größere Schwankungsbreiten bezüglich der Performance relevanter Werkstoffeigenschaften auftreten. Dadurch können im Gleisbetrieb veränderte Eigenschaften mit erweiterter Schwankungsbreite in RCF-Beständigkeit (Rollkontaktermüdung), Verschleiß und Schweißbarkeit auftreten.

Die Erfindung betrifft somit ein Verfahren zur Herstellung von perlitischen Schienenstählen eines auf eine Schienengüte spezifizierten Härtefaktorbereichs, welcher durch eine Ziellegierung sichergestellt wird, wobei zumindest ein primärmetallurgisches Aggregat zu einer ersten Schmelze führt, wobei aus der ersten Schmelze zumindest eine Analyse von Gehalten der Legierungselemente durchgeführt wird, wobei Ist-Werte der Schmelzanalyse an ein Steuersystem geleitet werden, wobei zwischen Einfluss- und Kompensationselementen unterschieden wird, wobei Einflusselemente Elemente sind, welche einen Einfluss auf den Härtefaktor eines Endprodukts haben, und Kompensationselemente Elemente sind, die einen Einfluss auf den Härtefaktor haben und den Einfluss von Einflusselementen kompensieren, wobei die Größe des Einflusses der Ist-Werte aller Einfluss- und Kompensationselemente auf den Härtefaktor des Endprodukts bekannt ist, wobei der zumindest eine Ist-Wert bezüglich derjenigen Einfluss- und Kompensationselemente erfasst wird, welche einen Einfluss auf den Härtefaktor des Endprodukts haben, wobei zum Härtefaktor des Endprodukts zumindest ein Kompensationselement ermittelt wird, welches den Härtefaktor einstellt, wobei eines oder mehrere Kompensationselemente in die Schmelze in einer Menge zulegiert werden, welche den Härtefaktor in einen Zielbereich bringt und den Einfluss eines oder mehrerer Einflusselemente auf den Härtefaktor kompensiert, und wobei hierfür im Steuersystem zwei Komponenten verwendet werden: ein Prognosemodell und ein Korrekturmodell, wobei mit dem Prognosemodell der Einfluss der Einfluss- und Kompensationselemente auf den Härtefaktor des Endprodukts und mit dem Korrekturmodell korrigierte Zielwerte der Kompensationselemente berechnet werden, um den Härtefaktor in einen Zielbereich zu bringen

Die Menge der zulegierten Kompensationselemente kann im Sinne der Erfindung auch 0 betragen, wenn dies für die Kompensation der Wirkung bzw. des Einflusses der Einflusselemente für nötig erachtet wird.

Eine Weiterbildung sieht vor, dass die Legierung so eingestellt wird, dass das Endprodukt der Norm EN 13674 oder EN 14811 bezüglich des Gefüges entspricht.

Eine Weiterbildung sieht vor, dass auf die Stahlherstellung folgende Nachbehandlungsschritte bis zum Erhalt des Endprodukts unverändert durchgeführt werden.

Eine Weiterbildung sieht vor, dass die Analyse zumindest in der ersten Schmelze des letzten primärmetallurgischen Aggregates einer Erzeugungsroute, insbesondere ab einem Abstich von Rohstahl, vor oder nach einer ersten Zugabe von Legierungselementen und zumindest nach einer zweiten Zugabe von Legierungselementen durchgeführt werden.

Eine Weiterbildung sieht vor, dass die Analyse mittels Probenentnahme nach EN ISO 14284 durchgeführt wird.

Eine Weiterbildung sieht vor, dass, wenn ein erstes Element durch einen Einsatzstoff in einem Anteil eingebracht wird, der unterhalb eines gewünschten, durch die Ziellegierung definierten Zielbereichs liegt, das erste Element, sofern es ein gewünschtes Legierungselement ist, in dem Umfang zulegiert wird, der zum Erreichen des Zielbereichs notwendig ist, oder, wenn das erste Element durch einen Einsatzstoff in einem Anteil eingebracht wird, der bereits im Zielbereich entspricht, nicht mehr zulegiert wird oder, wenn das erste Element durch einen Einsatzstoff in einem Anteil eingebracht wird, der über dem Zielbereich liegt, durch einen verringerten Legierungsanteil eines zweiten ähnlich wirkenden Elements oder einen erhöhten Legierungsanteil eines gegenläufig wirkenden dritten Elements kompensiert wird.

Eine Weiterbildung sieht vor, dass die Einflusselemente, deren Einfluss auf den Härtefaktor des Endprodukts kompensiert werden soll, eines, mehrere oder alle aus der Gruppe von Cu, Mo, Ni, Sn umfassen.

Eine Weiterbildung sieht vor, dass die Einflusselemente, deren Einfluss auf den Härtefaktor des Endprodukts kompensiert werden soll, Cu, Mo, Ni und/oder Sn umfassen.

Eine Weiterbildung sieht vor, dass als Kompensationselemente eines, mehrere oder alle aus der Gruppe von C, Si, Mn, Cr, V gewählt werden.

Eine Weiterbildung sieht vor, dass als Kompensationselemente C, Si, Mn, Cr und/oder V gewählt werden.

Eine Weiterbildung sieht vor, dass das Prognose- und/oder Korrekturmodell unter Verwendung eines, mehrerer oder aller nachfolgender Modelle erzeugt wird: lineare Regressionsmodelle, polynomiale Regressionsmodelle, Decision-Tree-Based Regressionsmodelle, Random-Forest-Regressionsmodelle, Nearest-Neighbour-Modelle, Neuronal-Network-Modelle, Support-Vector-Machine-Modelle, ADA-Boost-Modelle, Regression-Boost-Modelle, HIST-Boost-Modelle, XGBOOST-Modelle, Generalized-Additive-Modelle, Symbolic-Regression-Modelle.

Eine Weiterbildung sieht vor, dass das Prognose- und/oder Korrekturmodell unter Verwendung von linearen Regressionsmodellen, polynomialen Regressionsmodellen, Decision-Tree-Based Regressionsmodellen, Random-Forest-Regressionsmodellen, Nearest-Neighbour-Modellen, Neuronal-Network-Modellen, Support-Vector-Machine-Modellen, ADA-Boost-Modellen, Regression-Boost-Modellen, HIST-Boost-Modellen, XGBOOST-Modellen, Generalized-Additive-Modellen und/oder Symbolic-Regression-Modellen erzeugt wird.

Eine Weiterbildung sieht vor, dass, wenn der Härtefaktor betrachtet wird, der zumindest eine erfasste Ist-Wert der Analyse unter Heranziehung der Quadratwurzel der mittleren quadratischen Abweichung des Härtefaktors dem zumindest einen Prognosemodell und/oder Korrekturmodell zugeordnet wird, wobei für den zumindest einen Ist-Wert die Quadratwurzel der mittleren quadratischen Abweichung des Härtefaktors für alle Prognosemodelle und/oder Korrekturmodelle ermittelt wird und basierend auf den Ergebnissen jenes Prognosemodell und/oder Korrekturmodell mit der höchsten Genauigkeit gewählt wird.

Eine Weiterbildung sieht vor, dass, wenn mehrere werkstoffkundliche Eigenschaften betrachtet werden, der zumindest eine erfasste Ist-Wert der Analyse unter Heranziehung des Mittelwerts und/oder der Summe der Quadratwurzeln der mittleren quadratischen Abweichungen der werkstoffkundlichen Eigenschaften dem zumindest einen Prognosemodell und/oder Korrekturmodell zugeordnet wird, wobei für den zumindest einen Ist-Wert der Mittelwert und/oder die Summe der Quadratwurzeln der mittleren quadratischen Abweichungen der werkstoffkundlichen Eigenschaften für alle Prognosemodelle und/oder Korrekturmodelle ermittelt wird und basierend auf den Ergebnissen jenes Prognosemodell und/oder Korrekturmodell mit der höchsten Genauigkeit gewählt wird.

Eine Weiterbildung sieht vor, dass im Prognosemodell die folgende Formel verwendet wird: $HF = \left[K+\left\{{\sum }_{i = 1}^{n} {\sum }_{j = 1}^{m} \left({a}_{i , j}\times {\left({A}_{i}\right)}^{j}\right)\right\}\right] \times y - z ,$ wobei
- HF: ein Härtefaktor in % ist,
- K: ein Basisfaktor ist,
- a_{i,j}: eine n x m Matrix mit Faktoren, welche empirisch und/oder datengetrieben für jedes Element Aᵢ bestimmt werden, ist,
- Aᵢ: eine n x 1 Matrix der Elemente in Gew.-% in der Reihung C, Mn, Cr, Si, Mo, Cu, Ni, Sn und V ist,
- n: die Anzahl der Elemente ist,
- m: die Potenz der Funktion, insbesondere 2 abbildet, und
- y, z: Normierungsfaktoren sind, um den Härtefaktor zwischen einer festgesetzten unteren und einer festgesetzten oberen Grenze zwischen 0 und 100% zu skalieren.

Eine Weiterbildung sieht vor, dass folgende Faktoren als Ausgangspunkte verwendet werden: $K = 42 , 5 ;$ ${a}_{i , j} = \left(\begin{matrix}326 , 29 & - 113 , 04 \\ 36 , 75 & 3 , 03 \\ 138 , 2 & - 100 , 02 \\ 197 , 87 & - 245 , 21 \\ 111 , 08 & 3728 , 02 \\ 69 , 92 & - 176 , 82 \\ 84 , 36 & - 444 , 17 \\ 346 , 39 & - 13112 , 26 \\ 83 , 06 & - 146 , 5\end{matrix}\right) ;$ $y = 0 , 6623 ;$ $z = 152 , 23 .$

Eine Weiterbildung sieht vor, dass für eine bestimmte Stahlsorte der Härtefaktor HF zwischen einer Untergrenze HFᵤ und einer Obergrenze HFₒ liegt, wobei HFᵤ = 0% einem Härtewert von 200 HB und HFₒ = 100% einem Härtewert von 480 HB, insbesondere 440 HB, insbesondere 400 HB entspricht.

Eine Weiterbildung sieht vor, dass das verwendete Korrekturmodell eine Reihung der unterschiedlichen Kompensationselementen nach auflösungs- und ausbringungsspezifischen sowie ökonomischen Gesichtspunkten festlegt.

Der Begriff "Auflösung" bezieht sich auf die Auflösung der Einsatzstoffe in der Schmelze. Dies bedeutet in diesem Zusammenhang, dass im Zweifel ein Element als Kompensationselement eher nicht berücksichtigt wird, wenn sein Auflösen in der Schmelze zu lange dauert und insbesondere den gesamten Prozess verzögern würde.

Der Begriff "Ausbringung" bezieht sich auf die Ausbeute.

Eine Weiterbildung sieht vor, dass die dynamisch korrigierten Gehalte der Kompensationselemente in Schrittweiten ermittelt werden, wobei die Schrittweiten 5-10% jenes Intervalls entsprechen, in dem das entsprechende Legierungselement herstellungsbedingt schwanken darf.

Dies stellt sicher, dass eine Übersteuerung vermieden wird und dass Verfahrenskosten möglichst niedrig gehalten werden.

Eine Weiterbildung sieht vor, dass die Schrittweiten für die Korrekturen von Mn 0,01 Gew.-% und für Cr 0,005 Gew.-% betragen.

Eine Weiterbildung sieht vor, dass die Ziellegierung die folgende Ziellegierungszusammensetzung in Gew.-% umfasst:

| | |
|---|---|
| C | 0,38-1,4 |
| Si | 0,1-1,2 |
| Mn | 0,2-1,8 |

zudem Eisen und unvermeidbare Verunreinigungen,
wobei lediglich optional eines, mehrere oder alle der folgenden Elemente in Gew.-% enthalten sind:

| | |
|---|---|
| Cr | 0,001-1,5 |
| Cu | 0,001-0,4 |
| Mo | 0,001-0,06 |
| Ni | 0,001-0,4 |
| V | 0,001-0,3 |
| Sn | 0,001-0,03. |

Eine Weiterbildung sieht vor, dass die Ziellegierung die folgende Ziellegierungszusammensetzung in Gew.-% umfasst:

| | |
|---|---|
| C | 0,38-1,4 |
| Si | 0,1-1,2 |
| Mn | 0,2-1,8 |

zudem Eisen und unvermeidbare Verunreinigungen,
wobei lediglich optional folgende Elemente in Gew.-% enthalten sind:

| | |
|---|---|
| Cr | 0,001-1,5 |
| Cu | 0,001-0,4 |
| Mo | 0,001-0,06 |
| Ni | 0,001-0,4 |
| V | 0,001-0,3 und/oder |
| Sn | 0,001-0,03. |

Zudem betrifft die Erfindung ein Steuersystem zur Herstellung von perlitischen Schienenstählen eines auf eine Schienengüte spezifizierten Härtefaktorbereichs, welcher durch eine Ziellegierung sichergestellt wird, und zur Durchführung des erfindungsgemäßen Verfahrens, wobei das Steuersystem zwei Komponenten umfasst: ein Prognosemodell und ein Korrekturmodell, wobei das Prognosemodell ausgelegt ist, um den Einfluss von Einflusselementen und Kompensationselementen auf den Härtefaktor des Endprodukts zu berechnen und das Korrekturmodell ausgelegt ist, um korrigierte Zielwerte von Kompensationselementen zu berechnen, um den Härtefaktor in einen Zielbereich zu bringen.

Eine Weiterbildung sieht vor, dass das Prognose- und/oder Korrekturmodell unter Verwendung eines, mehrerer oder aller nachfolgender Modelle erzeugt wird: lineare Regressionsmodelle, polynomiale Regressionsmodelle, Decision-Tree-Based Regressionsmodelle, Random-Forest-Regressionsmodelle, Nearest-Neighbour-Modelle, Neuronal-Network-Modelle, Support-Vector-Machine-Modelle, ADA-Boost-Modelle, Regression-Boost-Modelle, HIST-Boost-Modelle, XGBOOST-Modelle, Generalized-Additive-Modelle, Symbolic-Regression-Modelle.

Eine Weiterbildung sieht vor, dass das Prognose- und/oder Korrekturmodell unter Verwendung von linearen Regressionsmodellen, polynomialen Regressionsmodellen, Decision-Tree-Based Regressionsmodellen, Random-Forest-Regressionsmodellen, Nearest-Neighbour-Modellen, Neuronal-Network-Modellen, Support-Vector-Machine-Modellen, ADA-Boost-Modellen, Regression-Boost-Modellen, HIST-Boost-Modellen, XGBOOST-Modellen, Generalized-Additive-Modellen und/oder Symbolic-Regression-Modellen erzeugt wird.

Eine Weiterbildung sieht vor, dass im Prognosemodell die folgende Formel verwendet wird: $HF = \left[K+\left\{{\sum }_{i = 1}^{n} {\sum }_{j = 1}^{m} \left({a}_{i , j}\times {\left({A}_{i}\right)}^{j}\right)\right\}\right] \times y - z ,$ wobei
- HF: ein Härtefaktor in % ist,
- K: ein Basisfaktor ist,
- a_{i,j}: eine n x m Matrix mit Faktoren, welche empirisch und/oder datengetrieben für jedes Element Aᵢ bestimmt werden, ist,
- Aᵢ: eine n x 1 Matrix der Elemente in Gew.-% in der Reihung C, Mn, Cr, Si, Mo, Cu, Ni, Sn und V ist,
- n: die Anzahl der Elemente ist,
- m: die Potenz der Funktion, insbesondere 2 abbildet, und
- y, z: Normierungsfaktoren sind, um den Härtefaktor zwischen einer festgesetzten unteren und einer festgesetzten oberen Grenze zwischen 0 und 100% zu skalieren.

Eine Weiterbildung sieht vor, dass folgende Faktoren als Ausgangspunkte verwendet werden: $K = 42,5 ;$ ${a}_{i , j} = \left(\begin{matrix}326 , 29 & - 113 , 04 \\ 36 , 75 & 3 , 03 \\ 138 , 2 & - 100 , 02 \\ 197 , 87 & - 245 , 21 \\ 111 , 08 & 3728 , 02 \\ 69 , 92 & - 176 , 82 \\ 84 , 36 & - 444 , 17 \\ 346 , 39 & - 13112 , 26 \\ 83 , 06 & - 146 , 5\end{matrix}\right) ;$ $y = 0 , 6623 ;$ $z = 152 , 23 .$

Eine Weiterbildung sieht vor, dass das verwendete Korrekturmodell die zu berechnenden Zielgehalte an unterschiedlichen Kompensationselementen nach auflösungs- und ausbringungsspezifischen sowie ökonomischen Gesichtspunkten reiht.

Eine Weiterbildung sieht vor, dass die dynamisch korrigierten Gehalte der Kompensationselemente in Schrittweiten ermittelt werden, wobei die Schrittweiten 5-10% jenes Intervalls entsprechen, in dem das entsprechende Legierungselement herstellungsbedingt schwanken darf.

Eine Weiterbildung sieht vor, dass die Ziellegierung die folgende Ziellegierungszusammensetzung in Gew.-% umfasst:

| | |
|---|---|
| C | 0,38-1,4 |
| Si | 0,1-1,2 |
| Mn | 0,2-1,8 |

zudem Eisen und unvermeidbare Verunreinigungen,
wobei lediglich optional eines, mehrere oder alle der folgenden Elemente in Gew.-% enthalten sind:

| | |
|---|---|
| Cr | 0,001-1,5 |
| Cu | 0,001-0,4 |
| Mo | 0,001-0,06 |
| Ni | 0,001-0,4 |
| V | 0,001-0,3 |
| Sn | 0,001-0,03. |

Eine Weiterbildung sieht vor, dass die Ziellegierung die folgende Ziellegierungszusammensetzung in Gew.-% umfasst:

| | |
|---|---|
| C | 0,38-1,4 |
| Si | 0,1-1,2 |
| Mn | 0,2-1,8 |

zudem Eisen und unvermeidbare Verunreinigungen,
wobei lediglich optional folgende Elemente in Gew.-% enthalten sind:

| | |
|---|---|
| Cr | 0,001-1,5 |
| Cu | 0,001-0,4 |
| Mo | 0,001-0,06 |
| Ni | 0,001-0,4 |

| | |
|---|---|
| V | 0,001-0,3 und/oder |
| Sn | 0,001-0,03. |

Die Erfindung betrifft ferner ein Gleisteil, insbesondere eine Schiene für Schienenfahrzeuge, aus einem Schienenstahl, hergestellt nach dem erfindungsgemäßen Verfahren.

Die Erfindung wird beispielhaft anhand einer Zeichnung dargestellt. Es zeigen dabei:
- Figur 1: den Prozessablauf des dynamischen Legierens;
- Figur 2: eine Auswertung der Modellvorhersagegenauigkeit;
- Figur 3: eine Zusammenfassung der chemischen Elemente und der Werkstoffeigenschaften des ersten Ausführungsbeispiels;
- Figur 4: eine Zusammenfassung der chemischen Elemente und der Werkstoffeigenschaften des zweiten Ausführungsbeispiels;
- Figur 5: eine Zusammenfassung der chemischen Elemente und der Werkstoffeigenschaften des dritten Ausführungsbeispiels;
- Figur 6: eine mögliche Gefügeausprägung bei Überschreitung eines kritischen Molybdängehaltes;
- Figur 7: eine Gefügeausprägung einer Standardschiene mit die Normgrenzen überschreitenden Gehalten an Einflusselementen.

Die Erfinder haben erkannt, dass es besonders vorteilhaft ist, zwischen Einfluss- und Kompensationselementen zu unterscheiden.

Die Wirkung bzw. der Einfluss der Einflusselemente wird durch das Zulegieren von Kompensationselementen kompensiert.

Die Wirkung bzw. der Einfluss, der in der Schmelze bereits vorhandenen Einflusselemente auf die werkstoffkundlichen Eigenschaften des Endprodukts wird mit dem Prognosemodell ermittelt. Eine gegebenenfalls notwendige Korrektur wird mit einem Korrekturmodell ermittelt.

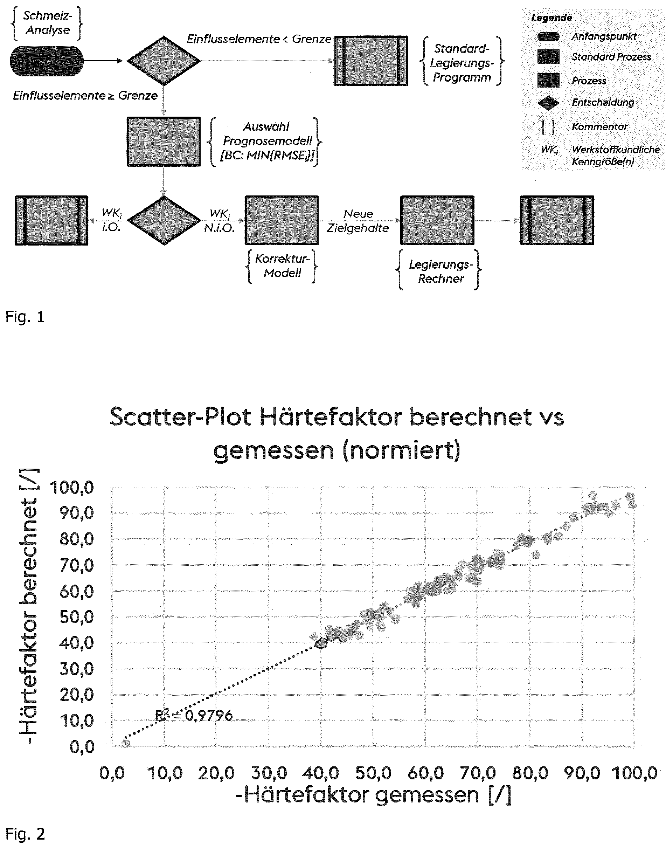

Figur 1 illustriert in vereinfachter Art und Weise das erfindungsgemäße Verfahren. Ausgehend von einer Schmelzanalyse wird entschieden, ob die Analyse innerhalb der spezifizierten Grenzen für die Einflusselementgehalte liegt oder sich davon unterscheidet. Wenn die Einflusselementgehalte innerhalb der Grenzen liegen, wird der Standard-Prozess durchgeführt, d.h. es kommt nicht zum dynamischen Legieren. Liegen die Einflusselementgehalte jedoch außerhalb der spezifizierten Grenzen, wird mittels des Prognosemodells der Einfluss auf zumindest eine spezifizierte werkstoffkundliche, insbesondere mechanische Eigenschaft ermittelt, um festzustellen, ob die prognostizierte Eigenschaft innerhalb oder außerhalb des durch die gewünschte Schienenstahlgüte definierten Zielbereichs liegt. Liegt die Eigenschaft im spezifizierten Zielbereich, wird der Standard-Prozess durchgeführt, d.h. es kommt nicht zum dynamischen Legieren. Wenn die prognostizierte Eigenschaft jedoch außerhalb des spezifizierten Zielbereichs liegt, werden mithilfe des Korrekturmodells adaptierte Legierungselementgehalte berechnet, sodass durch entsprechende Zugabe von Kompensationselementen die Wirkung der Einflusselemente dahingehend kompensiert werden kann, dass die betreffende Eigenschaft in den spezifizierten Zielbereich gebracht wird. Anschließend wird wieder nach dem Standard-Prozess verfahren. Nach dem dynamischen Legieren können gegebenenfalls auch erneut Analyse-Ist-Werte in das Prognosemodell eingespeist werden. Liegt die prognostizierte Eigenschaft im spezifizierten Zielbereich, wird nachfolgend nach dem Standard-Prozess verfahren. Liegt die prognostizierte Eigenschaft jedoch immer noch nicht im spezifizierten Zielbereich, kann erneut dynamisches Legieren durchgeführt werden.

In Figur 2 ist eine Auswertung der Modellvorhersagegenauigkeit dargestellt. Es ist ersichtlich, dass die gemessenen Werte des Härtefaktors HF mit den berechneten Werten stark korrelieren.

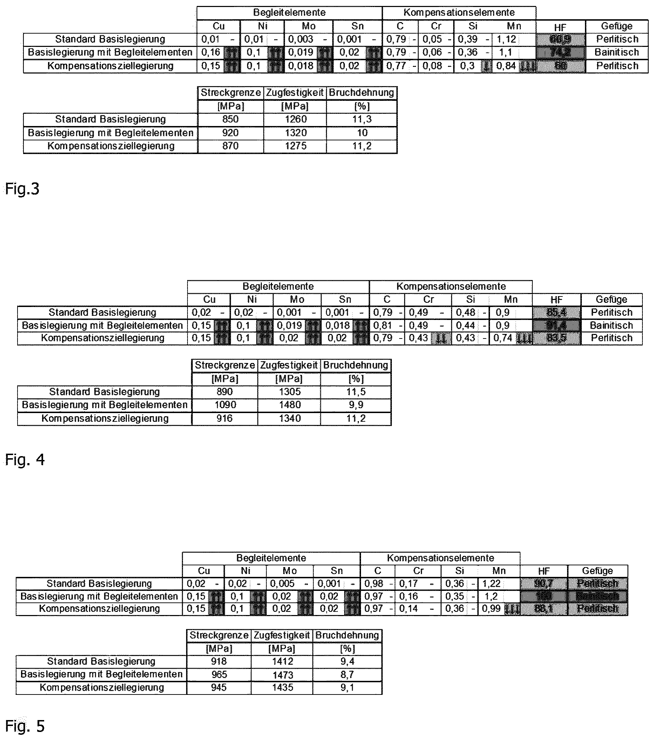

In Figur 3 sind Ergebnisse eines ersten Kompensationsversuchs dargestellt. Dieser Versuch umfasst eine eutektoide Standardschienengüte für den Mischverkehrsbereich mit und ohne Einflusselementfracht sowie den Abguß einer entsprechenden Kompensationslegierung auf Basis der Modellvorhersage. Es ist ersichtlich, dass durch die Erhöhung der Einflusselementfrachten ein wesentlich höherer Härtefaktor HF als bei der Standardbasislegierung erreicht wird. Darüber hinaus zeigt die Mikrostrukturanalyse das Auftreten von unerwünschten Fremdphasenanteilen (Bainit), welche unweigerlich mit dem erhöhten HF-Wert in dieser Schienenklasse korreliert.

Ein entsprechender Gegenlegierungsvorschlag auf Basis der Prognosemodellvorhersage wurde als Kompensationsziellegierung abgegossen, wobei hierbei die Zielgehalte an Mangan und Silizium durch das Kompensationsmodell zum Erhalt des ursprünglichen, der Basislegierung entsprechenden HF-Wertes vermindert wurden.

Andere leichte Schwankungen in der chemischen Analyse ergeben sich metallurgisch und messtechnisch bedingt und sind als nicht relevant einzustufen. Als Resultat wurden die negativen Effekte der Einflusselemente beseitigt, der HF-Wert wieder auf den gewünschten Standardwert der Basislegierung herabgesetzt und artgleiche, für den Gleisbetrieb taugliche Werkstoff- und Gefügeeigenschaften erhalten, womit der Kompensationsversuch als vollumfänglich positiv erachtet werden kann.

In Figur 4 sind Ergebnisse eines zweiten Kompensationsversich dargestellt. Dieser Versuch umfasst eine eutektoide Standardschienengüte mit erhöhtem Chromgehalt für Anwendungen in engen Bögen und im Schwerlastbereich mit und ohne Einflusselementfracht sowie den Abguß einer entsprechenden Kompensationslegierung auf Basis der Modellvorhersage. Es ist ersichtlich, dass durch die Erhöhung der Einflusselementfracht ein wesentlich höherer Härtefaktor HF als bei der Standardbasislegierung erreicht wird. Darüber hinaus zeigt die Mikrostrukturanalyse das Auftreten von unerwünschten Fremdphasenanteilen (Bainit), welche unweigerlich mit dem erhöhten HF-Wert in dieser Schienenklasse korreliert.

Ein entsprechender Gegenlegierungsvorschlag auf Basis der Prognosemodellvorhersage wurde als Kompensationsziellegierung abgegossen, wobei hierbei die Zielgehalte an Mangan und Chrom durch den Kompensationsalgorithmus zum Erhalt des ursprünglichen, der Basislegierung entsprechenden HF-Wertes vermindert wurden.

Andere leichte Schwankungen in der chemischen Analyse ergeben sich metallurgisch und messtechnisch bedingt und sind als nicht relevant einzustufen. Als Resultat wurden die negativen Effekte der Einflusselemente beseitigt, der HF-Wert wieder auf den gewünschten Standardwert der Basislegierung herabgesetzt und artgleiche, für den Gleisbetrieb taugliche Werkstoff- und Gefügeeigenschaften erhalten, womit der Kompensationsversuch als vollumfänglich positiv erachtet werden kann.

In Figur 5 sind Ergebnisse eines dritten Kompensationsversuchs dargestellt. Dieser Versuch umfasst eine übereutektoide Standardschienengüte mit erhöhten Kohlenstoffgehalten für Anwendungen unter höchsten Beanspruchungen im Schwerlastbereich mit und ohne Einflusselementfracht sowie den Abguss einer entsprechenden Kompensationslegierung auf Basis der Modellvorhersage. Es ist ersichtlich, dass durch die Erhöhung der Einflusselementfrachten ein wesentlich höherer Härtefaktor HF als bei der Standardbasislegierung erreicht wurde. Darüber hinaus zeigte die Mikrostrukturanalyse das Auftreten von unerwünschten Fremdphasenanteilen (Bainit), welche unweigerlich mit dem erhöhten HF-Wert in dieser Schienenklasse korreliert.

Ein entsprechender Gegenlegierungsvorschlag auf Basis der Modellvorhersage wurde als Kompensationsziellegierung abgegossen, wobei hierbei der Zielgehalt an Mangen durch den Kompensationsalgorithmus zum Erhalt des ursprünglichen, der Basislegierung entsprechenden HF-Wertes vermindert wurde.

Andere leichte Schwankungen in der chemischen Analyse ergeben sich metallurgisch und messtechnisch bedingt und sind als nicht relevant einzustufen. Als Resultat wurden die negativen Effekte der Einflusselemente beseitigt, der HF-Wert wieder auf den gewünschten Standardwert der Basislegierung herabgesetzt und artgleiche, für den Gleisbetrieb taugliche Werkstoff- und Gefügeeigenschaften erhalten, womit der Kompensationsversuch als vollumfänglich positiv erachtet werden kann.

Ist der Anteil an unerwünschten Einflusselementen zu hoch, kommt es zu Rissen im Endprodukt, welche im schlimmsten Fall, beispielsweise im Gleisbetrieb, zu Schienenbrüchen führen können.

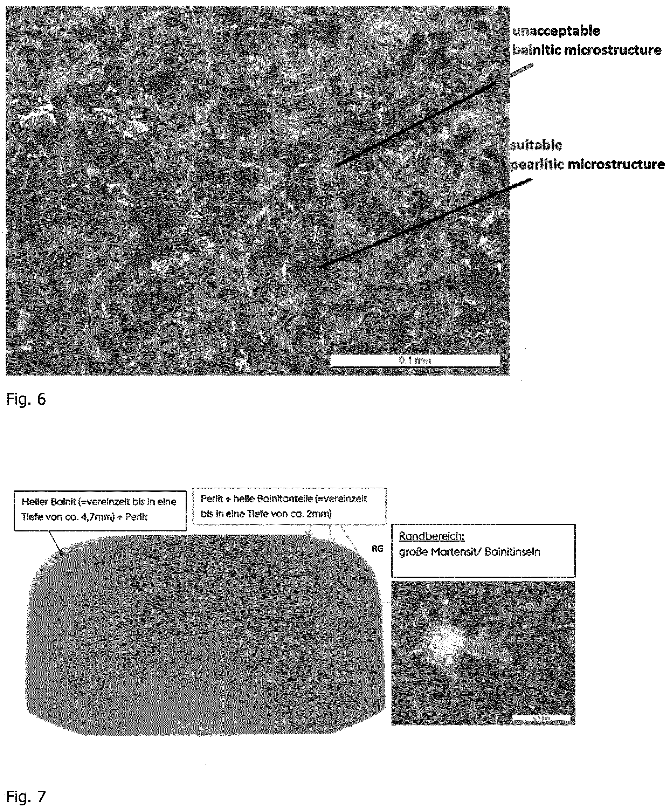

Ein zu hoher Molybdängehalt kann zu unerwünschter Gefügestruktur führen (Figur 6). Bei Überschreitung eines kritischen Molybdängehalts kann ein gemischtes Gefüge mit perlitischen und bainitischen Anteilen entstehen.

Die mögliche Auswirkung der Kombination der Einflusselemente kann mittels Stirnabschreckversuchen und großtechnischen Walzversuchen an fertigen Schienen untersucht werden.

Anhand der Stirnabschreckversuche zeigt sich eine signifikante Härtesteigerung um etwa 30 HB sowie einhergehend eine starke Verschiebung des Umwandlungsverhaltens zu kürzeren Zeiten bei Erhöhung der Einflusselementfracht auf zulässige Grenzen entsprechend EN 13674-1. Dies ist für den möglichen Prozessbereich der großtechnischen Produktion kritisch zu sehen, da das Auftreten von unerwünschten Fremdgefügebestandteilen gefördert wird. Ein weiteres Problem entsteht dadurch im Bereich von Chargenübergängen, da die chemische Analyse und somit die Gleichmäßigkeit der Werkstoffeigenschaften über die Schienenlänge verschlechtert wird.

Großtechnische Walzversuche einer mit Σ Cu + Mo + Ni = 0,28% legierten Standardschiene bestätigten weitgehend die Erwartungen aus den Stirnabschreckversuchen und zeigten die Möglichkeit unzulässiger Gefügeausbildung bei Überschreitung der gültigen Einflusselement-Normgrenzen in Kombination mit dem großtechnischen standardmäßigen Wärmebehandlungsprozess. Neben den zu erwartenden Bainitanteilen wurden durch die Normüberschreitung hierbei zusätzliche, unübliche Martensitphasen, welche auf ausgebildete Seigerungen rückzuführen sind, vorgefunden (Figur 7). Diese Martensitanteile stellen aufgrund ihres bruchfördernden Charakters mit Sicherheit einen kritischen Fall hinsichtlich Gefügeausprägung dar, und sind hinsichtlich sicherheitstechnischer Aspekte unbedingt zu vermeiden.

Mit dem vorliegenden Verfahren gelingt somit ein dynamisches und flexibles Legieren, bei welchem der Einfluss bzw. die Wirkung der Einflusselemente durch eine gezielte Zugabe der Kompensationselemente kompensiert wird.

Die Kompensationselemente können u.a. unter Berücksichtigung auflösungs- und ausbringungsspezifischer sowie ökonomischer Aspekte ausgewählt werden, sodass hierdurch die Kosteneffizienz des Gesamtverfahrens deutlich erhöht wird.

Außerdem gelingt es mit dem vorliegenden Verfahren, die gewünschten werkstoffkundlichen Eigenschaften trotz Verwendung von Einsatzstoffen mit erhöhter Einflusselementfracht sicher einzuhalten.

## Patentansprüche

1. Verfahren zur Herstellung von perlitischen Schienenstählen eines auf eine Schienengüte spezifizierten Härtefaktorbereichs, welcher durch eine Ziellegierung sichergestellt wird, wobei zumindest ein primärmetallurgisches Aggregat zu einer ersten Schmelze führt, wobei aus der ersten Schmelze zumindest eine Analyse von Gehalten der Legierungselemente durchgeführt wird, wobei Ist-Werte der Schmelzanalyse an ein Steuersystem geleitet werden,
**dadurch gekennzeichnet, dass**
zwischen Einfluss- und Kompensationselementen unterschieden wird, wobei
Einflusselemente Elemente sind, welche einen Einfluss auf den Härtefaktor eines Endprodukts haben, und Kompensationselemente Elemente sind, die einen Einfluss auf den Härtefaktor haben und den Einfluss von Einflusselementen kompensieren, wobei
die Größe des Einflusses der Ist-Werte aller Einfluss- und Kompensationselemente auf den Härtefaktor des Endprodukts bekannt ist, wobei
der zumindest eine Ist-Wert bezüglich derjenigen Einfluss- und Kompensationselemente erfasst wird, welche einen Einfluss auf den Härtefaktor des Endprodukts haben, wobei
zum Härtefaktor des Endprodukts zumindest ein Kompensationselement ermittelt wird, welches den Härtefaktor einstellt, wobei
eines oder mehrere Kompensationselemente in die Schmelze in einer Menge zulegiert werden, welche den Härtefaktor in einen Zielbereich bringt und den Einfluss eines oder mehrerer Einflusselemente auf den Härtefaktor kompensiert, und wobei
hierfür im Steuersystem zwei Komponenten verwendet werden: ein Prognosemodell und ein Korrekturmodell, wobei mit dem Prognosemodell der Einfluss der Einfluss- und Kompensationselemente auf den Härtefaktor des Endprodukts und mit dem Korrekturmodell korrigierte Zielwerte der Kompensationselemente berechnet werden, um den Härtefaktor in einen Zielbereich zu bringen.

2. Verfahren nach Anspruch 1, **dadurch gekennzeichnet, dass** die Legierung so eingestellt wird, dass das Endprodukt der Norm EN 13674 oder EN 14811 bezüglich des Gefüges entspricht.

3. Verfahren nach Anspruch 1 oder 2, **dadurch gekennzeichnet, dass** auf die Stahlherstellung folgende Nachbehandlungsschritte bis zum Erhalt des Endprodukts unverändert durchgeführt werden.

4. Verfahren nach einem der vorhergehenden Ansprüche, **dadurch gekennzeichnet, dass** die Analyse zumindest in der ersten Schmelze des letzten primärmetallurgischen Aggregates einer Erzeugungsroute, insbesondere ab einem Abstich von Rohstahl, vor oder nach einer ersten Zugabe von Legierungselementen und zumindest nach einer zweiten Zugabe von Legierungselementen durchgeführt werden.

5. Verfahren nach einem der vorhergehenden Ansprüche, **dadurch gekennzeichnet, dass** die Analyse mittels Probenentnahme nach EN ISO 14284 durchgeführt wird.

6. Verfahren nach einem der vorhergehenden Ansprüche, **dadurch gekennzeichnet, dass**, wenn ein erstes Element durch einen Einsatzstoff in einem Anteil eingebracht wird, der unterhalb eines gewünschten, durch die Ziellegierung definierten Zielbereichs liegt, das erste Element, sofern es ein gewünschtes Legierungselement ist, in dem Umfang zulegiert wird, der zum Erreichen des Zielbereichs notwendig ist, oder, wenn das erste Element durch einen Einsatzstoff in einem Anteil eingebracht wird, der bereits im Zielbereich entspricht, nicht mehr zulegiert wird oder, wenn das erste Element durch einen Einsatzstoff in einem Anteil eingebracht wird, der über dem Zielbereich liegt, durch einen verringerten Legierungsanteil eines zweiten ähnlich wirkenden Elements oder einen erhöhten Legierungsanteil eines gegenläufig wirkenden dritten Elements kompensiert wird.

7. Verfahren nach einem der vorhergehenden Ansprüche, **dadurch gekennzeichnet, dass** die Einflusselemente, deren Einfluss auf den Härtefaktor des Endprodukts kompensiert werden soll, eines, mehrere oder alle aus der Gruppe von Cu, Mo, Ni, Sn umfassen.

8. Verfahren nach einem der vorhergehenden Ansprüche, **dadurch gekennzeichnet, dass** als Kompensationselemente eines, mehrere oder alle aus der Gruppe von C, Si, Mn, Cr, V gewählt werden.

9. Verfahren nach einem der vorhergehenden Ansprüche, **dadurch gekennzeichnet, dass** das Prognose- und/oder Korrekturmodell unter Verwendung eines, mehrerer oder aller nachfolgender Modelle erzeugt wird: lineare Regressionsmodelle, polynomiale Regressionsmodelle, Decision-Tree-Based Regressionsmodelle, Random-Forest-Regressionsmodelle, Nearest-Neighbour-Modelle, Neuronal-Network-Modelle, Support-Vector-Machine-Modelle, ADA-Boost-Modelle, Regression-Boost-Modelle, HIST-Boost-Modelle, XGBOOST-Modelle, Generalized-Additive-Modelle, Symbolic-Regression-Modelle.

10. Verfahren nach einem der vorhergehenden Ansprüche, **dadurch gekennzeichnet, dass**, wenn der Härtefaktor betrachtet wird, der zumindest eine erfasste Ist-Wert der Analyse unter Heranziehung der Quadratwurzel der mittleren quadratischen Abweichung des Härtefaktors dem zumindest einen Prognosemodell und/oder Korrekturmodell zugeordnet wird, wobei für den zumindest einen Ist-Wert die Quadratwurzel der mittleren quadratischen Abweichung des Härtefaktors für alle Prognosemodelle und/oder Korrekturmodelle ermittelt wird und basierend auf den Ergebnissen jenes Prognosemodell und/oder Korrekturmodell mit der höchsten Genauigkeit gewählt wird.

11. Verfahren nach einem der vorhergehenden Ansprüche, **dadurch gekennzeichnet, dass**, wenn mehrere werkstoffkundliche Eigenschaften betrachtet werden, der zumindest eine erfasste Ist-Wert der Analyse unter Heranziehung des Mittelwerts und/oder der Summe der Quadratwurzeln der mittleren quadratischen Abweichungen der werkstoffkundlichen Eigenschaften dem zumindest einen Prognosemodell und/oder Korrekturmodell zugeordnet wird, wobei für den zumindest einen Ist-Wert der Mittelwert und/oder die Summe der Quadratwurzeln der mittleren quadratischen Abweichungen der werkstoffkundlichen Eigenschaften für alle Prognosemodelle und/oder Korrekturmodelle ermittelt wird und basierend auf den Ergebnissen jenes Prognosemodell und/oder Korrekturmodell mit der höchsten Genauigkeit gewählt wird.

12. Verfahren nach einem der vorhergehenden Ansprüche, **dadurch gekennzeichnet, dass** im Prognosemodell die folgende Formel verwendet wird: $HF = \left[K+\left\{{\sum }_{i = 1}^{n} {\sum }_{j = 1}^{m} \left({a}_{i , j}\times {\left({A}_{i}\right)}^{j}\right)\right\}\right] \times y - z ,$ wobei
HF ein Härtefaktor in % ist,
K ein Basisfaktor ist,
a_{i,j} eine n x m Matrix mit Faktoren, welche empirisch und/oder datengetrieben für jedes Element Aᵢ bestimmt werden, ist,
Aᵢ eine n x 1 Matrix der Elemente in Gew.-% in der Reihung C, Mn, Cr, Si, Mo, Cu, Ni, Sn und V ist,
n die Anzahl der Elemente ist,
m die Potenz der Funktion, insbesondere 2 abbildet, und
y, z Normierungsfaktoren sind, um den Härtefaktor zwischen einer festgesetzten unteren und einer festgesetzten oberen Grenze zwischen 0 und 100% zu skalieren.

13. Verfahren nach Anspruch 12, **dadurch gekennzeichnet, dass** folgende Faktoren als Ausgangspunkte verwendet werden: $K = 42 , 5 ;$ ${a}_{i , j} = \left(\begin{matrix}326 , 29 & - 113 , 04 \\ 36 , 75 & 3 , 03 \\ 138 , 2 & - 100 , 02 \\ 197 , 87 & - 245 , 21 \\ 111 , 08 & 3728 , 02 \\ 69 , 92 & - 176 , 82 \\ 84 , 36 & - 444 , 17 \\ 346 , 39 & - 13112 , 26 \\ 83 , 06 & - 146 , 5\end{matrix}\right) ;$ $y = 0 , 6623 ;$ $z = 152 , 23 .$

14. Verfahren nach einem der vorhergehenden Ansprüche, **dadurch gekennzeichnet, dass** für eine bestimmte Stahlsorte der Härtefaktor HF zwischen einer Untergrenze HFᵤ und einer Obergrenze HFₒ liegt, wobei HFᵤ = 0% einem Härtewert von 200 HB und HFₒ = 100% einem Härtewert von 480 HB, insbesondere 440 HB, insbesondere 400 HB entspricht.

15. Verfahren nach einem der vorhergehenden Ansprüche, **dadurch gekennzeichnet, dass** das verwendete Korrekturmodell eine Reihung der unterschiedlichen Kompensationselementen nach auflösungs- und ausbringungsspezifischen sowie ökonomischen Gesichtspunkten festlegt.

16. Verfahren nach einem der vorhergehenden Ansprüche, **dadurch gekennzeichnet, dass** die dynamisch korrigierten Gehalte der Kompensationselemente in Schrittweiten ermittelt werden, wobei die Schrittweiten 5-10% jenes Intervalls entsprechen, in dem das entsprechende Legierungselement herstellungsbedingt schwanken darf.

17. Verfahren nach einem der vorhergehenden Ansprüche, **dadurch gekennzeichnet, dass** die Ziellegierung die folgende Ziellegierungszusammensetzung in Gew.-% umfasst:
| | |
|---|---|
| C | 0,38-1,4 |
| Si | 0,1-1,2 |
| Mn | 0,2-1,8 |
zudem Eisen und unvermeidbare Verunreinigungen,
wobei lediglich optional eines, mehrere oder alle der folgenden Elemente in Gew.-% enthalten sind:
| | |
|---|---|
| Cr | 0,001-1,5 |
| Cu | 0,001-0,4 |
| Mo | 0,001-0,06 |
| Ni | 0,001-0,4 |
| V | 0,001-0,3 |
| Sn | 0,001-0,03. |

18. Steuersystem zur Herstellung von perlitischen Schienenstählen eines auf eine Schienengüte spezifizierten Härtefaktorbereichs, welcher durch eine Ziellegierung sichergestellt wird, und zur Durchführung des Verfahrens nach einem der vorhergehenden Ansprüche, **dadurch gekennzeichnet, dass** das Steuersystem zwei Komponenten umfasst: ein Prognosemodell und ein Korrekturmodell, wobei das Prognosemodell ausgelegt ist, um den Einfluss von Einflusselementen und Kompensationselementen auf den Härtefaktor des Endprodukts zu berechnen und das Korrekturmodell ausgelegt ist, um korrigierte Zielwerte von Kompensationselementen zu berechnen, um den Härtefaktor in einen Zielbereich zu bringen.

19. Steuersystem nach Anspruch 18, **dadurch gekennzeichnet, dass** das Prognose- und/oder Korrekturmodell unter Verwendung eines, mehrerer oder aller nachfolgender Modelle erzeugt wird: lineare Regressionsmodelle, polynomiale Regressionsmodelle, Decision-Tree-Based Regressionsmodelle, Random-Forest-Regressionsmodelle, Nearest-Neighbour-Modelle, Neuronal-Network-Modelle, Support-Vector-Machine-Modelle, ADA-Boost-Modelle, Regression-Boost-Modelle, HIST-Boost-Modelle, XGBOOST-Modelle, Generalized-Additive-Modelle, Symbolic-Regression-Modelle.

20. Steuersystem nach Anspruch 18 oder 19, **dadurch gekennzeichnet, dass** im Prognosemodell die folgende Formel verwendet wird: $HF = \left[K+\left\{{\sum }_{i = 1}^{n} {\sum }_{j = 1}^{m} \left({a}_{i , j}\times {\left({A}_{i}\right)}^{j}\right)\right\}\right] \times y - z ,$ wobei
HF ein Härtefaktor in % ist,
K ein Basisfaktor ist,
a_{i,j} eine n x m Matrix mit Faktoren, welche empirisch und/oder datengetrieben für jedes Element Aᵢ bestimmt werden, ist,
Aᵢ eine n x 1 Matrix der Elemente in Gew.-% in der Reihung C, Mn, Cr, Si, Mo, Cu, Ni, Sn und V ist,
n die Anzahl der Elemente ist,
m die Potenz der Funktion, insbesondere 2 abbildet, und
y, z Normierungsfaktoren sind, um den Härtefaktor zwischen einer festgesetzten unteren und einer festgesetzten oberen Grenze zwischen 0 und 100% zu skalieren.

21. Steuersystem nach Anspruch 20, **dadurch gekennzeichnet, dass** folgende Faktoren als Ausgangspunkte verwendet werden: $K = 42 , 5 ;$ ${a}_{i , j} = \left(\begin{matrix}326 , 29 & - 113 , 04 \\ 36 , 75 & 3 , 03 \\ 138 , 2 & - 100 , 02 \\ 197 , 87 & - 245 , 21 \\ 111 , 08 & 3728 , 02 \\ 69 , 92 & - 176 , 82 \\ 84 , 36 & - 444 , 17 \\ 346 , 39 & - 13112 , 26 \\ 83 , 06 & - 146 , 5\end{matrix}\right) ;$ $y = 0 , 6623 ;$ $z = 152 , 23 .$

22. Steuersystem nach einem der vorhergehenden Ansprüche 18 bis 21, **dadurch gekennzeichnet, dass** das verwendete Korrekturmodell die zu berechnenden Zielgehalte an unterschiedlichen Kompensationselementen nach auflösungs- und ausbringungsspezifischen sowie ökonomischen Gesichtspunkten reiht.

23. Steuersystem nach einem der vorhergehenden Ansprüche 18 bis 22, **dadurch gekennzeichnet, dass** die dynamisch korrigierten Gehalte der Kompensationselemente in Schrittweiten ermittelt werden, wobei die Schrittweiten 5-10% jenes Intervalls entsprechen, in dem das entsprechende Legierungselement herstellungsbedingt schwanken darf.

24. Steuersystem nach einem der vorhergehenden Ansprüche 18 bis 23, **dadurch gekennzeichnet, dass** die Ziellegierung die folgende Ziellegierungszusammensetzung in Gew.-% umfasst:
| | |
|---|---|
| C | 0,38-1,4 |
| Si | 0,1-1,2 |
| Mn | 0,2-1,8 |
zudem Eisen und unvermeidbare Verunreinigungen,
wobei lediglich optional eines, mehrere oder alle der folgenden Elemente in Gew.-% enthalten sind:
| | |
|---|---|
| Cr | 0,001-1,5 |
| Cu | 0,001-0,4 |
| Mo | 0,001-0,06 |
| | |
|---|---|
| Ni | 0,001-0,4 |
| V | 0,001-0,3 |
| Sn | 0,001-0,03. |

25. Gleisteil, insbesondere Schiene für Schienenfahrzeuge, aus einem Schienenstahl, hergestellt nach dem Verfahren nach einem der Ansprüche 1 bis 17.

## Geänderte Patentansprüche

### Geänderte Patentansprüche gemäss Regel 137(2) EPÜ.

1. Verfahren zur Herstellung von perlitischen Schienenstählen eines auf eine Schienengüte spezifizierten Härtefaktorbereichs, welcher durch eine Ziellegierung sichergestellt wird, wobei zumindest ein primärmetallurgisches Aggregat zu einer ersten Schmelze führt, wobei aus der ersten Schmelze zumindest eine Analyse von Gehalten der Legierungselemente durchgeführt wird, wobei Ist-Werte der Schmelzanalyse an ein Steuersystem geleitet werden,
**dadurch gekennzeichnet, dass**
zwischen Einfluss- und Kompensationselementen unterschieden wird, wobei
Einflusselemente Elemente sind, welche einen Einfluss auf den Härtefaktor eines Endprodukts haben, und Kompensationselemente Elemente sind, die einen Einfluss auf den Härtefaktor haben und den Einfluss von Einflusselementen kompensieren, wobei die Einflusselemente, deren Einfluss auf den Härtefaktor des Endprodukts kompensiert werden soll, eines, mehrere oder alle aus der Gruppe von Cu, Mo, Ni, Sn umfassen, wobei
als Kompensationselemente eines, mehrere oder alle aus der Gruppe von C, Si, Mn, Cr, V gewählt werden, wobei
die Größe des Einflusses der Ist-Werte aller Einfluss- und Kompensationselemente auf den Härtefaktor des Endprodukts bekannt ist, wobei
der zumindest eine Ist-Wert bezüglich derjenigen Einfluss- und Kompensationselemente erfasst wird, welche einen Einfluss auf den Härtefaktor des Endprodukts haben, wobei
zum Härtefaktor des Endprodukts zumindest ein Kompensationselement ermittelt wird, welches den Härtefaktor einstellt, wobei
eines oder mehrere Kompensationselemente in die Schmelze in einer Menge zulegiert werden, welche den Härtefaktor in einen Zielbereich bringt und den Einfluss eines oder mehrerer Einflusselemente auf den Härtefaktor kompensiert, und wobei hierfür im Steuersystem zwei Komponenten verwendet werden: ein Prognosemodell und ein Korrekturmodell, wobei mit dem Prognosemodell der Einfluss der Einfluss- und Kompensationselemente auf den Härtefaktor des Endprodukts und mit dem Korrekturmodell korrigierte Zielwerte der Kompensationselemente berechnet werden, um den Härtefaktor in einen Zielbereich zu bringen.

2. Verfahren nach Anspruch 1, **dadurch gekennzeichnet, dass** die Legierung so eingestellt wird, dass das Endprodukt der Norm EN 13674 oder EN 14811 bezüglich des Gefüges entspricht.

3. Verfahren nach Anspruch 1 oder 2, **dadurch gekennzeichnet, dass** auf die Stahlherstellung folgende Nachbehandlungsschritte bis zum Erhalt des Endprodukts unverändert durchgeführt werden.

4. Verfahren nach einem der vorhergehenden Ansprüche, **dadurch gekennzeichnet, dass** die Analyse zumindest in der ersten Schmelze des letzten primärmetallurgischen Aggregates einer Erzeugungsroute, insbesondere ab einem Abstich von Rohstahl, vor oder nach einer ersten Zugabe von Legierungselementen und zumindest nach einer zweiten Zugabe von Legierungselementen durchgeführt werden.

5. Verfahren nach einem der vorhergehenden Ansprüche, **dadurch gekennzeichnet, dass** die Analyse mittels Probenentnahme nach EN ISO 14284 durchgeführt wird.

6. Verfahren nach einem der vorhergehenden Ansprüche, **dadurch gekennzeichnet, dass**, wenn ein erstes Element durch einen Einsatzstoff in einem Anteil eingebracht wird, der unterhalb eines gewünschten, durch die Ziellegierung definierten Zielbereichs liegt, das erste Element, sofern es ein gewünschtes Legierungselement ist, in dem Umfang zulegiert wird, der zum Erreichen des Zielbereichs notwendig ist, oder, wenn das erste Element durch einen Einsatzstoff in einem Anteil eingebracht wird, der bereits im Zielbereich entspricht, nicht mehr zulegiert wird oder, wenn das erste Element durch einen Einsatzstoff in einem Anteil eingebracht wird, der über dem Zielbereich liegt, durch einen verringerten Legierungsanteil eines zweiten ähnlich wirkenden Elements oder einen erhöhten Legierungsanteil eines gegenläufig wirkenden dritten Elements kompensiert wird.

7. Verfahren nach einem der vorhergehenden Ansprüche, **dadurch gekennzeichnet, dass** das Prognose- und/oder Korrekturmodell unter Verwendung eines, mehrerer oder aller nachfolgender Modelle erzeugt wird: lineare Regressionsmodelle, polynomiale Regressionsmodelle, Decision-Tree-Based Regressionsmodelle, Random-Forest-Regressionsmodelle, Nearest-Neighbour-Modelle, Neuronal-Network-Modelle, Support-Vector-Machine-Modelle, ADA-Boost-Modelle, Regression-Boost-Modelle, HIST-Boost-Modelle, XGBOOST-Modelle, Generalized-Additive-Modelle, Symbolic-Regression-Modelle.

8. Verfahren nach einem der vorhergehenden Ansprüche, **dadurch gekennzeichnet, dass**, wenn der Härtefaktor betrachtet wird, der zumindest eine erfasste Ist-Wert der Analyse unter Heranziehung der Quadratwurzel der mittleren quadratischen Abweichung des Härtefaktors dem zumindest einen Prognosemodell und/oder Korrekturmodell zugeordnet wird, wobei für den zumindest einen Ist-Wert die Quadratwurzel der mittleren quadratischen Abweichung des Härtefaktors für alle Prognosemodelle und/oder Korrekturmodelle ermittelt wird und basierend auf den Ergebnissen jenes Prognosemodell und/oder Korrekturmodell mit der höchsten Genauigkeit gewählt wird.

9. Verfahren nach einem der vorhergehenden Ansprüche, **dadurch gekennzeichnet, dass**, wenn mehrere werkstoffkundliche Eigenschaften betrachtet werden, der zumindest eine erfasste Ist-Wert der Analyse unter Heranziehung des Mittelwerts und/oder der Summe der Quadratwurzeln der mittleren quadratischen Abweichungen der werkstoffkundlichen Eigenschaften dem zumindest einen Prognosemodell und/oder Korrekturmodell zugeordnet wird, wobei für den zumindest einen Ist-Wert der Mittelwert und/oder die Summe der Quadratwurzeln der mittleren quadratischen Abweichungen der werkstoffkundlichen Eigenschaften für alle Prognosemodelle und/oder Korrekturmodelle ermittelt wird und basierend auf den Ergebnissen jenes Prognosemodell und/oder Korrekturmodell mit der höchsten Genauigkeit gewählt wird.

10. Verfahren nach einem der vorhergehenden Ansprüche, **dadurch gekennzeichnet, dass** im Prognosemodell die folgende Formel verwendet wird: $HF = \left[K+\left\{{\sum }_{i = 1}^{n} {\sum }_{j = 1}^{m} \left({a}_{i , j}\times {\left({A}_{i}\right)}^{j}\right)\right\}\right] \times y - z ,$ wobei
HF ein Härtefaktor in % ist,
K ein Basisfaktor ist,
a_{i,j} eine n x m Matrix mit Faktoren, welche empirisch und/oder datengetrieben für jedes Element Aᵢ bestimmt werden, ist,
Aᵢ eine n x 1 Matrix der Elemente in Gew.-% in der Reihung C, Mn, Cr, Si, Mo, Cu, Ni, Sn und V ist,
n die Anzahl der Elemente ist,
m die Potenz der Funktion, insbesondere 2 abbildet, und
y, z Normierungsfaktoren sind, um den Härtefaktor zwischen einer festgesetzten unteren und einer festgesetzten oberen Grenze zwischen 0 und 100% zu skalieren.

11. Verfahren nach Anspruch 10, **dadurch gekennzeichnet, dass** folgende Faktoren als Ausgangspunkte verwendet werden:
K = 42,5; ${a}_{i , j} = \left(\begin{matrix}326 , 29 & - 113 , 04 \\ 36 , 75 & 3 , 03 \\ 138 , 2 & - 100 , 02 \\ 197 , 87 & - 245 , 21 \\ 111 , 08 & 3728 , 02 \\ 69 , 92 & - 176 , 82 \\ 84 , 36 & - 444 , 17 \\ 346 , 39 & - 13112 , 26 \\ 83 , 06 & - 146 , 5\end{matrix}\right) ;$
y = 0,6623;
z = 152,23.

12. Verfahren nach einem der vorhergehenden Ansprüche, **dadurch gekennzeichnet, dass** für eine bestimmte Stahlsorte der Härtefaktor HF zwischen einer Untergrenze HFᵤ und einer Obergrenze HFₒ liegt, wobei HFᵤ = 0% einem Härtewert von 200 HB und HFₒ = 100% einem Härtewert von 480 HB, insbesondere 440 HB, insbesondere 400 HB entspricht.

13. Verfahren nach einem der vorhergehenden Ansprüche, **dadurch gekennzeichnet, dass** das verwendete Korrekturmodell eine Reihung der unterschiedlichen Kompensationselementen nach auflösungs- und ausbringungsspezifischen sowie ökonomischen Gesichtspunkten festlegt.

14. Verfahren nach einem der vorhergehenden Ansprüche, **dadurch gekennzeichnet, dass** die dynamisch korrigierten Gehalte der Kompensationselemente in Schrittweiten ermittelt werden, wobei die Schrittweiten 5-10% jenes Intervalls entsprechen, in dem das entsprechende Legierungselement herstellungsbedingt schwanken darf.

15. Verfahren nach einem der vorhergehenden Ansprüche, **dadurch gekennzeichnet, dass** die Ziellegierung die folgende Ziellegierungszusammensetzung in Gew.-% umfasst:
| | |
|---|---|
| C | 0,38-1,4 |
| Si | 0,1-1,2 |
| Mn | 0,2-1,8 |
zudem Eisen und unvermeidbare Verunreinigungen,
wobei lediglich optional eines, mehrere oder alle der folgenden Elemente in Gew.-% enthalten sind:
| | |
|---|---|
| Cr | 0,001-1,5 |
| Cu | 0,001-0,4 |
| Mo | 0,001-0,06 |
| Ni | 0,001-0,4 |
| V | 0,001-0,3 |
| Sn | 0,001-0,03. |

16. Steuersystem zur Herstellung von perlitischen Schienenstählen eines auf eine Schienengüte spezifizierten Härtefaktorbereichs, welcher durch eine Ziellegierung sichergestellt wird, und zur Durchführung des Verfahrens nach einem der vorhergehenden Ansprüche, **dadurch gekennzeichnet, dass** das Steuersystem zwei Komponenten umfasst: ein Prognosemodell und ein Korrekturmodell, wobei das Prognosemodell ausgelegt ist, um den Einfluss von Einflusselementen und Kompensationselementen auf den Härtefaktor des Endprodukts zu berechnen und das Korrekturmodell ausgelegt ist, um korrigierte Zielwerte von Kompensationselementen zu berechnen, um durch Zulegieren zumindest eines Kompensationselements den Härtefaktor in einen Zielbereich zu bringen.

17. Steuersystem nach Anspruch 16, **dadurch gekennzeichnet, dass** das Prognose-und/oder Korrekturmodell unter Verwendung eines, mehrerer oder aller nachfolgender Modelle erzeugt wird: lineare Regressionsmodelle, polynomiale Regressionsmodelle, Decision-Tree-Based Regressionsmodelle, Random-Forest-Regressionsmodelle, Nearest-Neighbour-Modelle, Neuronal-Network-Modelle, Support-Vector-Machine-Modelle, ADA-Boost-Modelle, Regression-Boost-Modelle, HIST-Boost-Modelle, XGBOOST-Modelle, Generalized-Additive-Modelle, Symbolic-Regression-Modelle.

18. Steuersystem nach Anspruch 16 oder 17, **dadurch gekennzeichnet, dass** im Prognosemodell die folgende Formel verwendet wird: $HF = \left[K+\left\{{\sum }_{i = 1}^{n} {\sum }_{j = 1}^{m} \left({a}_{i , j}\times {\left({A}_{i}\right)}^{j}\right)\right\}\right] \times y - z ,$ wobei
HF ein Härtefaktor in % ist,
K ein Basisfaktor ist,
a_{i,j} eine n x m Matrix mit Faktoren, welche empirisch und/oder datengetrieben für jedes Element Aᵢ bestimmt werden, ist,
Aᵢ eine n x 1 Matrix der Elemente in Gew.-% in der Reihung C, Mn, Cr, Si, Mo, Cu, Ni, Sn und V ist,
n die Anzahl der Elemente ist,
m die Potenz der Funktion, insbesondere 2 abbildet, und
y, z Normierungsfaktoren sind, um den Härtefaktor zwischen einer festgesetzten unteren und einer festgesetzten oberen Grenze zwischen 0 und 100% zu skalieren.

19. Steuersystem nach Anspruch 18, **dadurch gekennzeichnet, dass** folgende Faktoren als Ausgangspunkte verwendet werden:
K = 42,5; ${a}_{i , j} = \left(\begin{matrix}326 , 29 & - 113 , 04 \\ 36 , 75 & 3 , 03 \\ 138 , 2 & - 100 , 02 \\ 197 , 87 & - 245 , 21 \\ 111 , 08 & 3728 , 02 \\ 69 , 92 & - 176 , 82 \\ 84 , 36 & - 444 , 17 \\ 346 , 39 & - 13112 , 26 \\ 83 , 06 & - 146 , 5\end{matrix}\right) ;$
y = 0,6623;
z = 152,23.

20. Steuersystem nach einem der vorhergehenden Ansprüche 16 bis 19, **dadurch gekennzeichnet, dass** das verwendete Korrekturmodell die zu berechnenden Zielgehalte an unterschiedlichen Kompensationselementen nach auflösungs- und ausbringungsspezifischen sowie ökonomischen Gesichtspunkten reiht.

21. Steuersystem nach einem der vorhergehenden Ansprüche 16 bis 20, **dadurch gekennzeichnet, dass** die dynamisch korrigierten Gehalte der Kompensationselemente in Schrittweiten ermittelt werden, wobei die Schrittweiten 5-10% jenes Intervalls entsprechen, in dem das entsprechende Legierungselement herstellungsbedingt schwanken darf.

22. Steuersystem nach einem der vorhergehenden Ansprüche 16 bis 21, **dadurch gekennzeichnet, dass** die Ziellegierung die folgende Ziellegierungszusammensetzung in Gew.-% umfasst:
| | |
|---|---|
| C | 0,38-1,4 |
| Si | 0,1-1,2 |
| Mn | 0,2-1,8 |
zudem Eisen und unvermeidbare Verunreinigungen,
wobei lediglich optional eines, mehrere oder alle der folgenden Elemente in Gew.-% enthalten sind:
| | |
|---|---|
| Cr | 0,001-1,5 |
| Cu | 0,001-0,4 |
| Mo | 0,001-0,06 |
| Ni | 0,001-0,4 |
| V | 0,001-0,3 |
| Sn | 0,001-0,03. |

23. Gleisteil, insbesondere Schiene für Schienenfahrzeuge, aus einem Schienenstahl, hergestellt nach dem Verfahren nach einem der Ansprüche 1 bis 15.
